(19) 

**Europäisches Patentamt**
**European Patent Office**
**Office européen des brevets**

(11) **EP 4 082 516 B1**

(12) **EUROPEAN PATENT SPECIFICATION**

(45) Date of publication and mention
of the grant of the patent:
**09.04.2025 Bulletin 2025/15**

(21) Application number: **20908121.5**

(22) Date of filing: **27.11.2020**

(51) International Patent Classification (IPC):
*A61K 6/887* (2020.01)     *A61K 6/84* (2020.01)
*A61K 6/17* (2020.01)       *A61K 6/71* (2020.01)
*A61K 6/16* (2020.01)

(52) Cooperative Patent Classification (CPC):
**A61K 6/17; A61K 6/16; A61K 6/71; A61K 6/887**

(86) International application number:
**PCT/JP2020/044221**

(87) International publication number:
**WO 2021/131490 (01.07.2021 Gazette 2021/26)**

(54) **DENTAL FILLING AND REPAIRING MATERIAL KIT**

ZAHNFÜLLUNG UND REPARATURMATERIALKIT

KIT DE MATÉRIAUX D'OBTURATION ET DE RÉPARATION DENTAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.12.2019 JP 2019231724**

(43) Date of publication of application:
**02.11.2022 Bulletin 2022/44**

(73) Proprietor: **Tokuyama Dental Corporation
Tokyo 110-0016 (JP)**

(72) Inventors:
• **AKIZUMI, Hironobu
Tokyo 110-0016 (JP)**
• **MORISAKI, Hiroshi
Tokyo 110-0016 (JP)**
• **MATSUO, Takuma
Tokyo 110-0016 (JP)**

(74) Representative: **HGF
HGF Europe LLP
Neumarkter Straße 18
81673 München (DE)**

(56) References cited:
EP-A1- 2 977 040      EP-A1- 3 366 269
WO-A1-2014/050634     WO-A1-2015/125470
WO-A1-2017/069274     WO-A1-2018/101236
JP-A- 2008 260 720    JP-A- 2012 153 640
JP-A- 2020 037 534    JP-A- 2020 090 574
JP-A- H09 255 516     JP-B2- 5 274 164

• **KNARVANG TORBJORN: "Mixing of
experimental composites | NIOM", NIOM
NEWSLETTER JANUARY 2014, 29 January 2014
(2014-01-29), Oslo, Norway, XP055905279,
Retrieved from the Internet <URL:https://niom.
no/mixing-of-experimental-composites/>
[retrieved on 20220325]**

Remarks:
The file contains technical information submitted
after the application was filed and not included in this
specification

EP 4 082 516 B1

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**Description**

TECHNICAL FIELD

The present invention relates to a dental filling restorative material kit.

BACKGROUND ART

A dental composite resin (hereinafter also referred to as "CR") is one type of material for restoring teeth damaged by dental caries, fractures, and the like, and is formed of a curable composition containing a polymerizable monomer, an inorganic filler and/or an organic filler. Restoration using the CR (CR restoration) has rapidly come into widespread use because, for example, the amount of tooth substance to be cut can be reduced, a color tone equivalent to natural teeth can be provided, and the operation thereof is easily performed. In recent years, as a result of the enhancement of mechanical strength, the enhancement of adhesive force to teeth, and the like, the dental composite resin has been used not only for the restoration of anterior teeth, but also for molar teeth to which high occlusal pressure is applied.

Although as described above, one of the excellent characteristics of the CR restoration is that highly aesthetic restoration can be performed, natural teeth are formed of dentine and enamel, and each part has different color tone (hue, color chroma, and value). In order to perform highly aesthetic restoration, it is necessary to take careful measures according to the states of teeth which are restored (teeth to be restored). For example, even if teeth to be restored have light damage, and shallow cavity, it is common practice to prepare a plurality of types of CRs having different color tones and to select the one that best matches the color tones of the actual teeth to be restored and its adjacent teeth (hereinafter, also referred to as "periphery of the teeth to be restored") (see, for example, Non-Patent Document 1). Furthermore, when a cavity is deep, since the tone of tooth can be seen in a state rich in gradation by fusing not only the tone of the tooth surface portion (enamel portion) but also the tone of the transparent deep layer portion (dentin portion), this delicate color tone can be reproduced by laminating and filling curable paste by changing the color tone for each depth.

In order to meet such demands, there have been proposed restorative material kits in which color tones are adjusted by changing the types and the amounts to be blended of coloring substances such as pigment substances and dye substances (see, for example, Patent Document 1), and restorative material kits for reproducing color tone by controlling a refractive index of polymer of the polymerizable monomer blended in restorative materials for enamel and dentine (see, for example, Patent Document 2).

However, the dental curable compositions prepared using such coloring substances such as pigment substances and dye substances may fade or discolor as time elapses after the restoration due to aged deterioration of coloring substances, and the appearance of the restored part may become incompatible with the natural teeth.

On the other hand, as a technology in which coloring is performed without use of coloring substances such as pigment substances and dye substances, there is a technology using a structural color, that is, a technology in which color production is performed by utilization of the reflection, interference, scattering, transmission, and the like, of light by fine particles in a medium. A technology is also known in which the technology described above is applied to produce the desired color of a composite material where inorganic particles are dispersed in a medium such as a resin (see, for example, Patent Documents 3 and 4).

For example, Patent Document 3 discloses that in "a fine particle-dispersed product where first fine particles having an average particle diameter in a range of 50 nm to 1 $\mu$m and having a Cv value of a particle diameter of 10% or less are dispersed in a medium, where the arrangement structure of the first fine particles in the dispersed product has an amorphous structure and where a short-range order structure satisfying a specific condition defined by a radial distribution function g(r) within a plane is provided", the arrangement structure of the fine particles is stably maintained, light of a specific wavelength can be reflected, and the angle dependence of the reflected light in which the peak wavelength of the reflected light changes with a change in the incident angle of the light can be sufficiently reduced.

Patent Document 4 discloses a curable composition, which is composed of, for example, "a curable composition containing a polymerizable monomer component (A), a spherical filler (B) having an average particle diameter within a range of 230 nm to 1000 nm and a polymerization initiator (C), in which 90% or more of individual particles constituting the spherical filler (B) are present within a range of plus or minus 5% of the average particle diameter and satisfying a condition where the refractive index $n_F$ at 25°C of the spherical filler (B) is higher than the refractive index $n_P$ at 25°C of a polymer obtained by polymerizing the polymerizable monomer component (A)", and which is "a curable composition, wherein when measurement is made for the curable composition in a state of having formed a cured article having a thickness of 1 mm, using a color difference meter, the cured article of the curable composition gives out a colored light having a value (V) of less than 5 and a chroma (C) of 0.05 or greater in the colorimetric values according to the Munsell Color System on a black background, and having a value (V) of 6 or greater and a chroma (C) of less than 2 in the colorimetric values according to the Munsell Color System on a white background". Patent Document 4 then discloses that a CR formed of the curable composition described above has excellent characteristics in which (1) since dye substances or pigment substances are not used, the problem of chronological discoloration is unlikely to occur, (2) the cured product thereof can be colored yellow to red, which are the identical colors as dentin colors (according to the average particle diameter of the spherical filler used), and moreover, (3) since the cured product has moderate transparency, the colors are easily harmonized with the colors of restored teeth, and without complicated shade taking and shade selection of the composite resin being performed, with one type of composite resin, the appearance of the restored teeth having a wide range of colors can be restored close to the appearance of natural teeth.

Patent Document 1: Japanese Patent No. 5436723
Patent Document 2: Japanese Patent No. 6258919
Patent Document 3: Japanese Patent No. 5274164
Patent Document 4: Japanese Patent No. 6250245
Patent Document 5: Japanese Patent No. 6732257
Patent Document 6: Japanese Patent No. 6732259

Non-Patent Document 1: "Adhesion YEARBOOK 2006", edited by Hideo Matsumura and Junji Tagami, first edition, Quintessence Publishing Co., Ltd., August, 2006, p. 129-137
Non-Patent Document 2: "Science & Technique of Composite Resin Restoration", written by Masashi Miyazaki, first edition, Quintessence Publishing Co., Ltd., January, 2010, p. 48-49

DISCLOSURE OF THE INVENTION

Problems to be Solved by the Invention

It is found from Patent Document 3 that the fine particles having a uniform particle diameter are dispersed so as to achieve an amorphous structure as a whole while having a specific short-range order structure, and that thus a structural color of a constant color tone can be produced without being affected by a change in the incident angle of light. Also in the description of Patent Document 4, colored light caused by interference of the cured product of the curable composition (or a CR formed of the curable composition) is produced in a portion in which the particles thereof are accumulated relatively regularly, and colored light caused by scattering is produced in a portion in which the particles thereof are dispersed randomly, and thus it can be inferred that even in this system, a balance between longdistance irregularity and short-distance regularity in the dispersed state of the spherical filler is important in order to obtain the effects described above.
However, in the CR disclosed in Patent Document 4, as the spherical filler (B), only one type of "aggregate including inorganic spherical particles having a predetermined average primary particle diameter within a range of 230 nm to 1000 nm in which in a number-based particle size distribution of the aggregate, 90% or more of all particles are present within a range of plus or minus 5% of the predetermined average primary particle diameter" is used. Therefore, it is not clear what influences are exerted on the effects described above when a plurality of aggregates having different average primary particle diameters are used. Furthermore, it is found that depending on conditions under which individual components are kneaded so as to prepare the CR, even at an extremely low frequency, desired effects are not obtained.
The present inventors have proposed a curable composition in which inorganic spherical particles are dispersed in a specific dispersion state as a curable composition securely providing a composite material being like a cured product of a curable composition disclosed in Patent Document 4 and being capable of exhibiting the above-mentioned effect even in a case where a plurality of spherical filler aggregates is used (see, Patent Documents 5 and 6). In other words, Patent Documents 5 and 6 have proposed a curable composition (hereinafter, also referred to as a "structural color developing curable composition") containing a polymerizable monomer and inorganic spherical particles, wherein the inorganic spherical particles have a specific average particle diameter and a specific particle size distribution, and in which a dispersion state of the inorganic spherical particles in the curable composition is defined by a radial distribution function $g(r)$ of the cured product based on the findings found by the present inventors that the curable composition securely can provide a cured product developing a structural color having a predetermined color tone can be produced without being affected by a change of the incident angle of light when "the conditions related to a constituting component" in which the refractive index and a refractive index of the polymerizable monomer satisfy a specific size relation, and "the conditions related to a state of composition" in which inorganic spherical particles in the curable composition are dispersed so as to satisfy the specific condition are both satisfied at the same time. Note here that the reason why the dispersion state of the inorganic spherical particles of the curable composition is defined by the radial distribution function $g(r)$ of the cured product is because the dispersion state of the inorganic spherical particles in the cured product directly reflects the dispersion state of a composition before being cured.
With the above-mentioned structural color development curable composition, even when a fine filler is added, for example, in order to adjust the viscosity of a curable composition such as a CR before being cured and the contrast ratio of a cured product, it is possible to obtain the same effects as the cured product of the curable composition disclosed in Patent Document 2. Specifically, it is possible to obtain excellent effects in which (1) since dye substances or pigment substances are not used, the problem of chronological discoloration is unlikely to occur, (2) the cured product thereof can be colored in desired color tones within a wide range of color tones from a bluish transparent color tone to the color tones of yellow to red, which are the same colors as dentin colors (according to the average particle diameter of a spherical filler used), and moreover, (3) since the cured product can be made to have moderate transparency, when the cured product is used as a dental restorative material, the colors are easily harmonized with the colors of teeth to be restored, and without complicated shade taking and shade selection of the composite resin being performed, with one type of composite resin, the appearance of the teeth to be restored having a wide range of colors can be restored close to the appearance of natural teeth. The structural color development curable composition is used as CR, cavities having deep dentine is restored, the effect as expected can be obtained.

However, when CR including the above-mentioned structural color development curable composition is used for restoring a cavity of class III (a cavity adjacent to the anterior tooth and not containing an incisal angle) or a class IV cavity (a cavity adjacent to the anterior tooth and containing an incisal angle) in which there is no dentin in the deep layer, a restored portion may appear black (the developed structural color cannot be recognized visually). This is assumed to be because the transparency of the cured product is so high that reflected light and scattered light are difficult to reach the observer.

In order to solve the problem which is specific to CR including the structural color development curable composition and which has not been recognized so far, the present invention has an object to provide a dental filling restorative material kit capable of obtaining high color tone compatibility even when it is used for restoration of cavities of class III and class IV.

Means for Solving the Problems

The present inventors have thought that the above-mentioned problems can be dissolved when a cured product of a polymerization curable composition having low transparency is disposed for a foundation layer (a portion corresponding to a vicinity of the rear side of the teeth to be restored), and a cured product of a polymerization curable composition having structural color developing property is disposed on a surface exposed layer (the front side of the teeth to be restored), and have performed thorough studies. As a result, the present inventors have found that when value of the polymerization curable composition for the foundation layer is set to a predetermined value, the problems are dissolved, and thereby have completed the present invention.

In other words, the dental filling restorative material kit according to the present invention is a dental filling restorative material kit for restoring a tooth having a cavity, the kit including a first polymerization curable composition (A) for forming a surface exposed layer to be exposed to a surface after restoration, and a second polymerization curable composition (B) for forming a foundation layer serving as a foundation of the surface exposed layer.

The first polymerization curable composition (A) satisfies the following (a1) to (a3):

(a1) the first polymerization curable composition (A) contains a first polymerizable monomer component, inorganic particles, and a first polymerization initiator component;
(a2) the inorganic particles satisfy all of the following conditions (i) to (iii):

(i) the inorganic particles include an identical particle diameter spherical particle group (G-PID) which is formed of an aggregate of inorganic spherical particles having a predetermined average primary particle diameter within a range of 100 nm to 1000 nm and in which 90% or more of all the particles are present within a range of plus or minus 5% of the predetermined average primary particle diameter, in a number-based particle size distribution of the aggregate; and the number of the identical particle diameter spherical particle groups included in the inorganic particles is one or more;
(ii) when the number of the identical particle diameter spherical particle groups included in the inorganic particles is represented as a, and each of the identical particle diameter spherical particle groups is represented as $G\text{-}PID_m$ (when a is 1, m is 1 and when a is 2 or more, m is a natural number from 1 to a) in ascending order of the average primary particle diameters thereof, the average primary particle diameters of each $G\text{-}PID_m$ differ from each other by 25 nm or more; and
(iii) when a refractive index of a cured product of the first polymerizable monomer component at 25°C with respect to light of a wavelength of 589 nm is represented as $n_{(MX)}$, and a refractive index of the inorganic spherical particles constituting each $G\text{-}PID_m$ at 25°C with respect to light of a wavelength of 589 nm is represented as $n_{(G\text{-}PIDm)}$, for any $n_{(G\text{-}PIDm)}$, a relationship of $n_{(MX)} < n_{(G\text{-}PIDm)}$ is satisfied; and

(a3) in a cured product (A') obtained by curing the first polymerization curable composition (A), when a radial distribution function representing a probability of finding an inorganic spherical particle at a distance of r away from a center of a given inorganic spherical particle is defined by the following formula (1):

$$g(r) = \{1/\langle\rho\rangle\} \times \{dn/da\} \quad (1),$$

wherein $\langle\rho\rangle$ represents an average particle density of the inorganic spherical particles in an observation plane, dn represents the number of the inorganic spherical particles being present in a region between a circle at a distance of r and r + dr away from a given inorganic spherical particle within the observation plane,

da represents an area of the region (da = $2\pi r \cdot dr$), and

$\langle\rho\rangle$, dn, and da are determined based on a scanning electron microscope image in which a plane in the cured product (A') is the observation plane, and

a radial distribution function graph representing a relationship between $r/r_0$ and g(r) corresponding to r at that time is created by plotting $r/r_0$ on x-axis and g(r) on y-axis, wherein $r/r_0$ is a dimensionless number standardized by dividing a distance r away from a center of a given inorganic spherical particle dispersed in the cured product (A') by an average particle diameter $r_0$ of all the inorganic spherical particles dispersed in the cured product (A'), the inorganic spherical particles are dispersed in the first polymerization curable composition (A) such that the cured product (A') has a short-range order structure satisfying the following conditions (I) and (II):

(I) a closest particle-to-particle distance $r_1$ defined as r corresponding to a peak top of a peak closest to an origin among peaks appearing in the radial distribution function graph is a value being 1 to 2 times the average particle diameter $r_0$ of all the inorganic spherical particles dispersed in the cured product; and

(II) when r corresponding to a peak top of a peak second closest to the origin among the peaks appearing in the radial distribution function graph is represented as a second closest particle-to-particle distance $r_2$, a local minimum value of the radial distribution function g(r) between the closest particle-to-particle distance $r_1$ and the second closest particle-to-particle distance $r_2$ is a value of 0.56 to 1.10.

The second polymerization curable composition (B) satisfies the following (b1) and (b2):

(b1) the second polymerization curable composition (B) contains a second polymerizable monomer component, a colorant component, and a second polymerization initiator component, and

(b2) the colorant component is blended such that value (V) of a colorimetric value by a Munsell color system of colored light measured using a color-difference meter in a black background is 5 or more with respect to a sample including a cured product (B') of the second polymerization curable composition (B) and having a thickness of 1 mm.

Preferably, the first polymerization curable composition (A) further includes a superfine particle group (G-SFP) having an average primary particle diameter of less than 100 nm, and having an average primary particle diameter being smaller than the average primary particle diameter of $G\text{-}PID_1$ by 25 nm or more, as inorganic particles. The total amount of the identical particle diameter spherical particle groups dispersed in the first polymerization curable composition (A) and an amount of the superfine particle group are 10 parts by mass to 1500 parts by mass, and 0.1 parts by mass to 50 parts by mass, respectively, with respect to 100 parts by mass of the first polymerizable monomer component.

Preferably, the first polymerization curable composition (A) does not substantially contain a colorant component. Herein, the colorant component means a pigment and/or a dye substance, and does not include the identical particle diameter spherical particle group (and the superfine particle group when the superfine particle group is included).

Furthermore, preferably, the average primary particle diameter of all the identical particle diameter spherical particle groups included in the first polymerization curable composition (A) is in a range of 230 nm to 1000 nm, and, preferably, the first polymerization curable composition (A) includes only one type of identical particle diameter spherical particle group having an average primary particle diameter of the inorganic spherical particles within a range from 230 nm to 350 nm. Furthermore, preferably, $\Delta n$ defined by ($n_{(G\text{-}PIDm)} - n_{(MX)}$), a difference between $n_{(MX)}$ and $n_{(G\text{-}PIDm)}$, is 0.001 to 0.1 with respect to any $n_{(G\text{-}PIDm)}$.

The dental filling restorative material kit according to the present invention is for restoring a cavity of class III and/or a cavity of class IV.

Effects of the Invention

In the dental filling restorative material kit according to the present invention, since a polymerizable monomer composition (first polymerization curable composition) for a surface exposed layer in which coloring substances such as a dye substance and a pigment substance are not used can be disposed in a surface layer, there is no fading problem, and therefore, it is possible to exhibit the effect that the color tone can be harmonized with the surroundings by the structural color. Furthermore, since polymerization curable composition (second polymerization curable composition) for a foundation layer having appropriate value is used, restoration of a lost portion of the anterior tooth, in particular, also in restoration of a cavity of class III or a cavity of class IV, restoration having compatibility with the color tone of high natural teeth can be performed by a simple operation.

BRIEF DESCRIPTION OF THE DRAWINGS

Fig. 1A is a view showing an example of a scanning electron microscope image of an observation plane in a cured product of Reference Example 1.
Fig. 1B is a view showing an example of coordinate data obtained from the scanning electron microscope image of Fig. 1A.
Fig. 2 is a diagram showing a radial distribution function graph for g(r) calculated based on a parameter determined from the coordinate data of Fig. 1B.
Fig. 3 is a diagram showing a radial distribution function graph for a cured product of Reference Example 2.
Fig. 4 is a diagram showing a radial distribution function graph for a cured product of Reference Example 3.
Fig. 5 is a diagram showing a radial distribution function graph for a cured product of Reference Example 4.
Fig. 6 is a diagram showing a radial distribution function graph for a cured product of Reference Comparative Example 2.

PREFERRED MODE FOR CARRYING OUT THE

A dental filling restorative material kit according to the present embodiment is a dental filling restorative material kit for repairing a tooth having a cavity, the kit including a first polymerization curable composition (A) for forming a surface exposed layer to be exposed to a surface after restoration, and a second polymerization curable composition (B) for forming a foundation layer serving as a foundation of the surface exposed layer.
The first polymerization curable composition (A) satisfies the following (a1) to (a3):

(a1) the first polymerization curable composition (A) contains a first polymerizable monomer component, inorganic particles, and a first polymerization initiator component;
(a2) the inorganic particles satisfy all of the following conditions (i) to (iii):

(i) the inorganic particles include an identical particle diameter spherical particle group (G-PID) which is formed of an aggregate of inorganic spherical particles having a predetermined average primary particle diameter within a range of 100 nm to 1000 nm and in which 90% or more of all the particles are present within a range of plus or minus 5% of the predetermined average primary particle diameter in a number-based particle size distribution of the aggregate; and the number of the identical particle diameter spherical particle groups included in the inorganic particles is one or more;
(ii) when the number of the identical particle diameter spherical particle groups included in the inorganic particles is represented as a, and each of the identical particle diameter spherical particle groups is represented as G-PID$_m$ (when a is 1, m is 1 and when a is 2 or more, m is a natural number from 1 to a) in ascending order of the average primary particle diameters thereof, the average primary particle diameters of each G-PID$_m$ differ from each other by 25 nm or more; and
(iii) when a refractive index of a cured product of the first polymerizable monomer component at 25°C with respect to light of a wavelength of 589 nm is represented as $n_{(MX)}$, and a refractive index of the inorganic spherical particles constituting each G-PID$_m$ at 25°C with respect to light of a wavelength of 589 nm is represented as $n_{(G\text{-}PIDm)}$, for any $n_{(G\text{-}PIDm)}$, a relationship of $n_{(MX)} < n_{(G\text{-}PIDm}$ is satisfied; and

(a3) in a cured product (A') obtained by curing the first polymerization curable composition (A), when a radial distribution function representing a probability of finding an inorganic spherical particle at a distance of r away from a center of a given inorganic spherical particle is defined by the following formula (1):

$$g(r) = \{1/\langle\rho\rangle\} \times \{dn/da\} \ (1),$$

wherein $\langle\rho\rangle$ represents an average particle density of the inorganic spherical particles in an observation plane, dn represents the number of the inorganic spherical particles being present in a region between a circle at a distance of r and r + dr away from a given inorganic spherical particle within the observation plane,

da represents an area of the region (da = $2\pi r \cdot dr$), and

$<\rho>$, dn, and da are determined based on a scanning electron microscope image in which a plane in the cured product (A') is the observation plane, and

a radial distribution function graph representing a relationship between $r/r_0$ and $g(r)$ corresponding to r at that time is created by plotting $r/r_0$ on x-axis and $g(r)$ on y-axis, wherein $r/r_0$ is a dimensionless number standardized by dividing a distance r away from a center of a given inorganic spherical particle dispersed in the cured product (A') by an average particle diameter $r_0$ of all the inorganic spherical particles dispersed in the cured product (A'), the inorganic spherical particles are dispersed in the first polymerization curable composition (A) such that the cured product (A') has a short-range order structure satisfying the following conditions (I) and (II):

(I) a closest particle-to-particle distance $r_1$ defined as r corresponding to a peak top of a peak closest to an origin among peaks appearing in the radial distribution function graph is a value being 1 to 2 times the average particle diameter $r_0$ of all the inorganic spherical particles dispersed in the cured product; and

(II) when r corresponding to a peak top of a peak second closest to the origin among the peaks appearing in the radial distribution function graph is represented as a second closest particle-to-particle distance $r_2$, a local minimum value of the radial distribution function $g(r)$ between the closest particle-to-particle distance $r_1$ and the second closest particle-to-particle distance $r_2$ is a value of 0.56 to 1.10.

The second polymerization curable composition (B) satisfies the following (b1) and (b2):

(b1) the second polymerization curable composition (B) contains a second polymerizable monomer component, a colorant component, and a second polymerization initiator component, and

(b2) the colorant component is blended such that value (V) of a colorimetric value by a Munsell color system of colored light measured using a color-difference meter in a black background is 5 or more with respect to a sample including a cured product (B') of the second polymerization curable composition (B) and having a thickness of 1 mm.

Herein, the first polymerization curable composition (A) corresponds to the structural color development curable composition described above. The presence of the cured product of the second polymerization curable composition (B) as a foundation of the cured product of the first polymerization curable composition (A) reduces the amount of light passing through the cured product (A') of the first polymerization curable composition (A) (structural color development curable composition) to an appropriate extent similar to dentin existing deep in the cavities. As a result, in the dental filling restorative material kit according to the present embodiment, even when being used for restoring a cavity of class III (a cavity adjacent to the anterior tooth and not containing an incisal angle) or a class IV cavity (a cavity adjacent to the anterior tooth and containing an incisal angle), excellent color tone compatibility can be exhibited.

A detailed description of the first polymerization curable composition (A) and the second polymerization curable composition (B) constituting the dental filling restorative material kit according to the present embodiment will be given below.

[1. First polymerization curable composition (A)]

The first polymerization curable composition (A) is a polymerization curable composition for forming a surface exposed layer to be exposed to a surface after restoration. The first polymerization curable composition (A) contains a first polymerizable monomer component and inorganic spherical particles as described above, and the inorganic spherical particles have a specific average particle diameter and a specific particle size distribution, and satisfies "the conditions related to a constituting component" (a1) and (a2) in which the refractive index and a refractive index of the cured product of the first polymerizable monomer component satisfy the specific size relation; and "conditions related to a state of composition" (a3) in which inorganic spherical particles are dispersed so as to satisfy the specific conditions in the polymerization curable composition at the same time, is a polymerization curable composition providing a cured product developing a structural color having a predetermined constant color without being affected by a change in the incident angle of light. Therefore, a surface exposed layer is formed of the cured product (A') of the first polymerization curable composition (A), the appearance of the teeth to be restored having a wide range of colors can be restored close to the appearance of natural teeth with one type of composite resin without complicated shade taking and shade selection of the composite resin being performed.

Note here that the first polymerization curable composition (A) is basically falls into the category of the curable composition disclosed in Patent Document 4, and is not particularly different from the curable composition disclosed in Patent Document 4, except that it can include a plurality of the identical particle diameter spherical particle groups (G-PID), and that when an inorganic filler which is one of "other additives" used as an optional component in Patent Document 4 is contained therein, a particle size which does not adversely affect the above effect is specified. However, the "conditions related to a state of composition" is not particularly recognized in Patent Document 4. Hereinafter, the conditions, also including the content disclosed in Patent Document 4, will be described.

[1-1. Condition (a1)]

A first polymerization curable composition (A) contains a first polymerizable monomer component, inorganic particles, and a polymerization initiator component.

<<First polymerizable monomer component>>

As the first polymerizable monomer component, it is possible to use the same component as the component to be usable as a polymerizable monomer in the curable composition disclosed in Patent Document 4. Examples of polymerizable monomers to be suitably used include 1,6-bis(methacrylethyloxycarbonylamino)trimethylhexane, triethylene glycol dimethacrylate, tetraethylene glycol dimethacrylate, neopentyl glycol dimethacrylate, 1,6-hexanediol dimethacrylate, 1,9-nonanediol dimethacrylate, 2,2-bis[(3-methacryloyloxy-2-hydroxypropyloxy)phenyl] propane, 2,2-bis(4-(methacryloyloxypolyethoxyphenyl) propane, pentaerythritol trimethacrylate, pentaerythritol tetramethacrylate, and the like.
As the first polymerizable monomer component, a plurality of types of polymerizable monomers are generally used in order to adjust the physical properties (mechanical properties and adhesion to a tooth substance in a dental application) of the resin matrix in the cured product (A') of the first polymerization curable composition (A). The types and the amounts of polymerizable monomers are such that the refractive index of the polymerizable monomer (mixture) falls within a range of 1.38 to 1.55 because the condition regarding the refractive index is easily satisfied. That is, when, according to the invention, a silica-titanium group element oxide-based composite oxide having a refractive index that is easily adjusted is used as the inorganic spherical particles, the refractive index thereof falls within a range of about 1.45 to 1.58 depending on the content of silica. And when the refractive index of the polymerizable monomer component is set to fall into a range of 1.38 to 1.55, the refractive index of the cured product to be obtained can be adjusted to fall into a range of about 1.40 to 1.57, and as a result, the condition described above can be easily satisfied. Note here that the refractive indexes of the polymerizable monomer and the cured product of the polymerizable monomer can be determined by measuring refractive index with respect to the sodium D line (wavelength: 589 nm) using an Abbe refractometer at 25°C.
Herein, "refractive index" means refractive index with respect to light of a wavelength of 589 nm at 25°C.

<<Inorganic particles>>

The inorganic particles are not particularly different from the inorganic particles disclosed in Patent Document 4, except that a plurality of identical particle diameter spherical particle groups (G-PID) can be included as described above, and that the particle size of the inorganic filler, which is one of the "other additives" used as an optional component in the curable composition disclosed in Patent Document 4, is specified when the inorganic filler is contained, in which case the particle size does not adversely affect the above effect is specified. In other words, the inorganic particles included in the first polymerization curable composition (A) include spherical particle groups having a predetermined average primary particle diameter in the range of 100 nm to 1000 nm, and the individual particles constituting the spherical particle groups are made of substantially the same substance, and need to include one or a plurality of the identical particle diameter spherical particle groups (G-PID) in which 90% (the number of particles) or more of the individual particles are present within a range of plus or minus 5% of the average primary particle diameter {condition (i)}. Furthermore, as necessary, the inorganic particles may include a superfine particle group (G-SFP) including inorganic particles having an average primary particle diameter being less than 100 nm and smaller than the smallest average primary particle diameter by 25nm or more among the average primary particle diameters of the identical particle diameter spherical particle group (G-PID).
The above-mentioned one or a plurality of conditions to be satisfied by the G-PID are described later as the description of the condition (a2). Herein, material of the inorganic spherical particles constituting the G-PID, and G-SFP are described.

<Material of the inorganic spherical particles constituting G-PID>

According to the invention, since the refractive index is easily adjusted, particles formed of silica-titanium group element oxide-based composite oxide (such as silica-zirconia and silica-titania) are used.
Here, the silica-titanium group element oxide-based composite oxide means particles mainly including a composite oxide of silica and a titanium group element (element of Group 4 in the periodic table) oxide, and the refractive index thereof is in a range of about 1.45 to 1.58 according to the content of silica. Specific examples of the silica-titanium group element oxide-based composite oxide includes silica-titania, silica-zirconia, silica-titania-zirconia, and the like. Among these, since high X-ray opacity can be also provided, silica-zirconia is preferable. Although the composite ratio of silica-zirconia is not particularly limited, since sufficient X-ray opacity is provided and the refractive index is made to fall into a suitable range which will be described later, it is preferable that the content of silica be 70 mol% to 95 mol% and that the content of the titanium group element oxide be 5 mol% to 30 mol%.

In the silica-titanium group element oxide-based composite oxide particles, compounding of a metal oxide other than silica and a titanium group element oxide is also allowed as long as the amount thereof is small. Specifically, an alkali metal oxide such as sodium oxide or lithium oxide may be contained as long as the content thereof is 10 mol% or less.

Although a method for producing the silica-titanium group element oxide-based composite oxide particles is not particularly limited, in order to obtain a spherical filler, for example, a so-called sol-gel method of adding a mixture solution including a hydrolyzable organosilicon compound and a hydrolyzable organotitanium group metal compound to an alkaline solvent, performing hydrolysis and precipitating a reaction product is suitably employed.

The inorganic spherical particles formed of these silica-titanium group element oxide-based composite oxide may be surface-treated with a silane coupling agent. The surface treatment is performed with the silane coupling agent, and thus when the inorganic spherical particles are formed into an organic-inorganic composite filler which will be described later, excellent interfacial strength with the organic resin matrix of the organic-inorganic composite filler is provided. Typical examples of the silane coupling agent include organosilicon compounds such as $\gamma$-methacryloyloxyalkyltrimethoxysilane and hexamethyldisilazane. The amount of surface treatment with the silane coupling agent is not particularly limited, and although an optimal value may be determined after the mechanical properties and the like of the obtained cured product are previously checked by experiments, an example of a suitable range is a range of 0.1 parts by mass to 15 parts by mass with respect to 100 parts by mass of the inorganic spherical particles.

The blending amount of the identical particle diameter spherical particle group G-PID is 10 parts by mass tc 1500 parts by mass with respect to 100 parts by mass of the first polymerizable monomer component in the total amount of all G-PID included (in other words, as the total amount of inorganic spherical particles). Since the cured product (A') of the first polymerization curable composition (A) has moderate transparency and the effect of developing a structural color is enhanced, the blending amount of G-PID is preferably 50 to 1500 parts by mass and more preferably 100 to 1500 parts by mass, with respect to 100 parts by mass of the first polymerizable monomer component. When a plurality of types of G-PID are included, with consideration given to the color tones of structural colors by each G-PID and a desired color tone in the cured product (A'), the blending amount of each G-PID is preferably allocated as necessary in the range into which the total content falls.

<Superfine particle group G-SFP>

The superfine particle group (G-SFP) is a particle aggregate formed with the inorganic particles having an average primary particle diameter of less than 100 nm, and is blended in order to, for example, adjust the viscosity of first polymerization curable composition (A), or to adjust the contrast ratio of the cured product (A') of the first polymerization curable composition (A). However, the average primary particle diameter of G-SFP needs to be smaller by 25 nm or more, than the average primary particle diameter ($d_1$) of G-PID$_1$ having an average primary particle diameter that is the smallest among the G-PID to be blended in the inorganic particles. When the condition as described above is not satisfied, the dispersed state of the inorganic spherical particles is adversely affected, and thus a structural color is unlikely to be developed. The shape of the inorganic particles of G-SFP is not particularly limited, and may be amorphous or spherical. The lower limit of the average primary particle diameter is normally 2 nm.

Since the development of a structural color is little affected, the average primary particle diameter of G-SFP is preferably 3 nm to 75 nm, and more preferably 5 nm to 50 nm. For the same reason, the average primary particle diameter of G-SFP is preferably smaller than the average primary particle diameter ($d_1$) of G-PID$_1$ by 30 nm or more, and more preferably by 40 nm or more.

As the material of the inorganic particles of G-SFP, the same material as that of the inorganic spherical particles can be used without particular limitation. As in the inorganic spherical particles, the surface treatment can be performed with the silane coupling agent. The suitable embodiment is basically the same as that of the inorganic spherical particles except for the average primary particle diameter and the shape.

The blending amount of superfine particle group G-SFP may be appropriately determined with consideration given to, for example, viscosity of the first polymerization curable composition (A) and the contrast ratio of the cured product (A') of the first polymerization curable composition (A), but the blending amount is usually 0.1 parts by mass to 50 parts by mass and suitably 0.2 parts by mass to 30 parts by mass with respect to 100 parts by mass of the first polymerizable monomer component.

<<First polymerization initiator component>>

As the first polymerization initiator component, it is possible to use the same component as the component to be usable as a polymerization initiator in the curable composition disclosed in Patent Document 4. When a dental direct filling restoration application in which curing is often performed within an oral cavity is assumed, as the first polymerization initiator component, a chemical polymerization initiator and/or a photopolymerization initiator is preferably used, and since a mixing operation is not necessary, the photopolymerization initiator is more preferably used.

As the chemical polymerization initiator, a chemical polymerization initiator which is formed of two or more components and in which a polymerization initiation species (radical) is generated when these components make contact with each other can be used without particular limitation. Examples of the chemical polymerization initiator include chemical polymerization initiators which are formed by various combinations of organic peroxides/amines, organic peroxides/amines/organic sulfinic acids, organic peroxides/amines/arylborates, arylborates/acidic compounds, barbituric acid derivatives/copper compounds/halogen compounds, and the like. Among them, in terms of ease of handling, organic peroxides/amines are preferable.

Examples of the organic peroxides include hydroperoxides, peroxyketals, ketone peroxides, alkylsilyl peroxides, diacyl peroxides, peroxyesters, and the like, which are known.

Sulfinic acids such as benzenesulfinic acid, p-toluenesulfinic acid and salts thereof; barbituric acids such as 5-butylbarbituric acid; and the like; may be mixed in the chemical polymerization initiator formed of organic peroxides/amines.

Examples of the photopolymerization initiator include benzoin alkyl ethers, benzyl ketals, benzophenones, $\alpha$-diketones, a thioxanthone compound, bisacylphosphine oxides, and the like. These photopolymerization initiators may be blended with a reducing agent such as tertiary amines, aldehydes or a sulfur-containing compound. Furthermore, a photoacid generator such as a diaryliodonium salt-based compound, a sulfonium salt-based compound, a sulfonate ester compound, a halomethyl-substituted-S-triazine derivative, and a pyridinium salt-based compound may be blended.

These polymerization initiators may be used singly, or two or more types thereof may be mixed so as to be used. As the amount of polymerization initiator mixed, an effective amount is preferably selected according to the purpose, but 0.01 to 10 parts by mass of the polymerization initiator is normally used, and 0.1 to 5 parts by mass is more preferably used, with respect to 100 parts by mass of the polymerizable monomer component.

[1-2. Condition (a2)]

Inorganic particles satisfy all of the following conditions (i) to (iii):

(i) the inorganic particles include an identical particle diameter spherical particle group (G-PID) including an aggregate of inorganic spherical particles having a predetermined average primary particle diameter within a range of 100 nm to 1000 nm and in which 90% or more of all the particles are present within a range of plus or minus 5% of the predetermined average primary particle diameter, in a number-based particle size distribution of the aggregate, and a number of the identical particle diameter spherical particle groups included in the inorganic particles is one or more;

(ii) when the number of the identical particle diameter spherical particle groups included in the inorganic particles is represented as a, and the identical particle diameter spherical particle groups are represented as $G\text{-PID}_m$ (when a is 1, m is 1 and when a is 2 or more, m is a natural number from 1 to a) in ascending order of the average primary particle diameters thereof, the average primary particle diameters of $G\text{-PID}_m$ differ from each other by 25 nm or more; and

(iii) when the refractive index of a cured product of the first polymerizable monomer component is represented as $n_{(MX)}$, and the refractive index of the inorganic spherical particles constituting each $G\text{-PID}_m$ is represented as $n_{(G\text{-PIDm})}$, for any $n_{(G\text{-PIDm})}$, a relationship of $n_{(MX)} < n_{(G\text{-PIDm})}$ holds true.

Hereinafter, these conditions will be described in detail.

<<Condition (i)>>

The identical particle diameter spherical particle group G-PID means an aggregate which includes inorganic spherical particles having a predetermined average primary particle diameter within a range of 100 nm to 1000 nm, and in which 90% or more of all the particles are present within the range of plus or minus 5% of the predetermined average primary particle diameter, in the number-based particle size distribution of the aggregate. The individual inorganic spherical particles of the aggregate are formed of substantially the same substance.

Herein, the average primary particle diameter of the inorganic spherical particles means an average value determined by taking a photograph of G-PID with a scanning electron microscope, selecting 30 or more particles observed within a unit field of view of the photograph, and obtaining an average of the respective primary particle diameters (maximum diameters) thereof. The term "spherical" may be "substantially spherical", and it is not always necessary to provide a completely true sphere. The photograph of G-PID is shot with the scanning electron microscope, the maximum diameters of respective particles (30 or more particles) within a unit field of view thereof are measured, the average uniformity is obtained by dividing particle diameters orthogonal to the maximum diameters by the maximum diameters, and the average uniformity is 0.6 or more and more preferably 0.8 or more.

In the cured product (A') of the first polymerization curable composition (A), each of the constituent particles of G-PID, which is the aggregate of the inorganic particles being spherical and having a narrow particle diameter distribution (number-based particle size distribution), have a specific short-range order structure so as to be dispersed in the resin matrix; and thus diffractive interference occurs according to Bragg conditions and light of a specific wavelength is emphasized, with the result that colored light of a color tone corresponding to the average primary particle diameter is produced (structural color is developed). In other words, in order to develop a structural color, 90% (pieces) or more of the inorganic spherical particles of G-PID need to be present within a range of plus or minus 5% of the average primary particle diameter. In order to develop a structural color having a specific color tone within a wide range of bluish-yellowish-reddish colors, the average primary particle diameter of the inorganic spherical particles of G-PID needs to fall within a range of 100 nm to 1000 nm. In a case where spherical particles having an average primary particle diameter of less than 100 nm are used, the phenomenon of interference of visible light is unlikely to occur, and a structural color is unlikely to be developed. On the other hand, in a case where spherical particles having an average primary particle diameter of greater than 1000 nm are used, though the development of the phenomenon of interference of light can be expected, it is not preferable to be used as a dental filling restorative material because the spherical particles settle down or the polishing property is lowered.

When the average primary particle diameter is 230 nm to 800 nm, a yellowish-reddish structural color (colored light) is easily developed, whereas when the average primary particle diameter is 150 nm or more and less than 230 nm, a bluish structural color (colored light) is easily developed.

Since a yellowish-reddish structural color (colored light) being preferable for a dental filling restorative material is developed, the average primary particle diameter of G-PID is preferably 230 nm to 800 nm, more preferably 240 nm to 500 nm and further preferably 260 nm to 350 nm. When G-PID having an average primary particle diameter of 230 nm or more and less than 260 nm is used, colored light obtained is yellowish, and the composite material is useful for the restoration of teeth in the class of B system (reddish yellow) in a shade guide ("VITA Classical", manufactured by Vita Zahnfabrik H. Rauter GmbH & Co. KG), and is particularly useful for the restoration of a cavity formed over from the enamel to the dentine. When G-PID having an average primary particle diameter in a range of 260 nm to 350 nm is used, colored light obtained is reddish, and the composite material is useful for the restoration of teeth in the class of A system (reddish brown) in the shade guide ("VITA Classical", manufactured by Vita Zahnfabrik H. Rauter GmbH & Co. KG), and is particularly useful for the restoration of a cavity formed over from the enamel to the dentine. Since the hue of the dentine is often reddish, when only G-PID having an average primary particle diameter in a range of 260 nm to 350 nm is used, the composite material is most preferable because compatibility with teeth to be restored having a variety of color tones is widely enhanced. On the other hand, when only G-PID having an average primary particle diameter of 150 nm or more and less than 230 nm is used, though colored light obtained is bluish, and the color tone compatibility with the tooth substance is likely to be poor in a cavity formed over from the enamel to the dentine, the composite material is useful for the restoration of the enamel and is particularly useful for the restoration of an incisal portion.

The number of types of G-PID included in the inorganic particles in the first polymerization curable composition (A) may be one or more. The number a of G-PID included is preferably 1 to 5, more preferably 1 to 3, and further preferably 1 or 2.

<<Condition (ii)>>

When a plurality of types of G-PID is included in the inorganic particles, the average primary particle diameters of each G-PID$_m$ need to differ from each other by 25 nm or more. In other words, when the number of G-PIDs included in the inorganic particles is represented by a (for example, a = 3), each G-PID is represented as G-PID$_m$ (when a is 1, m is 1 and when a is equal to or greater than 2, m is a natural number from 1 to a) in ascending order of the average primary particle diameters thereof, and the average primary particle diameter of each G-PID$_m$ is represented as d$_m$, each d$_m$ needs to differ from one another by 25 nm or more. For example, when a = 3, it is necessary that $|d_1 - d_2| \geq 25$ nm and $|d_2 - d_3| \geq 25$ nm (naturally, a relationship of $|d_1 - d_3| \geq 25$ nm is satisfied). When this condition is satisfied, for example, a specific structural color can be developed for each G-PID (according to the average primary particle diameter). It is inferred because, for example, each G-PID is dispersed in the form of a coagulated material in which a small number of inorganic spherical particles that do not exceed about 20 pieces are coagulated with very loose bonding force, and thus G-PID can be dispersed so as to have a short-range order structure capable of developing a structural color according to each G-PID. On the contrary, when this condition is not satisfied, a structural color is unlikely to be developed. It is considered that this is because the particle diameter distribution of all the inorganic spherical particles is broad, thus the inorganic spherical particles of each G-PID are substituted with each other so as to be dispersed and the same phenomenon occurs as in the case where an aggregate of a single type of inorganic spherical particles which do not satisfy the condition of the number-based particle size distribution is used.

When a plurality of types of G-PID is used in the first polymerization curable composition (A), the average primary particle diameters d$_m$ of each G-PID$_m$ preferably differ from each other by 30 nm or more, and more preferably differ from each other by 40 nm or more. In other words, a difference between $d_m$ and $d_{m-1}$ is preferably 30 nm or more, and more preferably 40 nm or more.

When a plurality of G-PIDs is included, since each G-PID has a very sharp particle size distribution and the difference described above is produced between the average primary particle diameters, the particle size distributions of G-PID are unlikely to overlap each other, and even when they partially overlap each other, the particle size distribution of each G-PID can be checked. In other words, the particle size distribution of the inorganic particles included in the first polymerization curable composition (A) has the same number of independent peaks as the number of G-PID included in the composite material in a range of 100 nm to 1000 nm, and even when a part of each of the peaks overlaps, waveform processing is performed so as to be able to check the average primary particle diameters of each G-PID and the number-based particle size distribution. The particle size distribution of the inorganic particles included in the first polymerization curable composition (A) can also be checked, for example, by performing image processing on an electron microscope photogram of an internal surface of the cured product (A') of the first polymerization curable composition (A).

<<Condition (iii)>>

In the first polymerization curable composition (A), when the refractive index of a cured product of the first polymerizable monomer component as a resin matrix at the time of curing is represented as $n_{(MX)}$, and the refractive index of the inorganic spherical particles constituting each G-PID$_m$ is represented as $n_{(G-PIDm)}$, for any $n_{(G-PIDm)}$, a following relationship:

$$n_{(MX)} < n_{(G-PIDm)}$$

needs to hold true. In a case where the relationship described above is not satisfied, even when a structural color is developed, light of a short wavelength is easily scattered in the resin matrix, and thus it is difficult to observe the developed structural color. From the viewpoint of visibility and clarity of the developed structural color and color tone compatibility when the composite material is used as a dental filling restorative material, for any $n_{(G-PIDm)}$, $\Delta n$ ($= n_{(G-PIDm)} - n_{(MX)}$) as a difference between $n_{(G-PIDm)}$ and $n_{(MX)}$ is preferably 0.001 to 0.1, more preferably 0.002 to 0.1, and further preferably 0.005 to 0.05.

As described above, the refractive index of the first polymerizable monomer component is set in a range of 1.38 to 1.55, and thus the refractive index ($n_{(MX)}$) of the cured product of the resin matrix can be made to fall into a range of 1.40 to 1.57. As described above, the content of silica is changed, and thus the refractive index ($n_{(G-PIDm)}$) of the silica-titanium group element oxide-based composite oxide can be changed in a range of about 1.45 to 1.58. Hence, these relationships are utilized, and thus $\Delta n$ can easily be made to fall into a suitable range.

[1-3. Condition (a3)]

Condition (a3) is a "condition related to a state of composition" in which inorganic spherical particles in the first polymerization curable composition (A) are dispersed so as to satisfy a specific condition. In condition (a3), from the reasons that a dispersion state of inorganic spherical particles of the cured product (A') of the first polymerization curable composition (A) directly reflects the dispersion state of the inorganic spherical particles in the polymerization-curable composition before curing; and that the dispersion state of inorganic spherical particles in the cured product (A') can be quantitatively grasped by analytical means such as an electron microscope, the dispersion state of the inorganic spherical particles in the first polymerization curable composition (A) is defined by the radial distribution function g(r) (more specifically, a radial distribution function graph) in the cured product (A').

Herein, the radial distribution function g(r) is a function representing a probability of finding an inorganic spherical particle at a distance of r away from the center of a given inorganic spherical particle in the cured product (A') obtained by curing the first polymerizable monomer composition (A), and is defined by the following formula (1):

$$g(r) = \{1/<\rho>\} \times \{dn/da\} \quad (1).$$

In the formula (1), $<\rho>$ represents an average particle density of the inorganic spherical particles in an observation plane, dn represents the number of the inorganic spherical particles being present in a region between a circle at a distance r and r + dr from a given inorganic spherical particle within the observation plane, da represents an area of the region (da = $2\pi r \cdot dr$), and $<\rho>$, dn, and da are determined based on a scanning electron microscope image in which a plane in the cured product (A') is the observation plane.

The radial distribution function graph represents the relationship between $r/r_0$ and g(r) corresponding to r at that time, and in the graph, dimensionless number ($r/r_0$) standardized by dividing the distance r from the center of a given inorganic spherical particle dispersed in the cured product (A') by the average particle diameter $r_0$ of all the inorganic spherical particles dispersed in the cured product (A') is on the x-axis, and the radial distribution function graph g(r) mentioned above is on the y-axis.

According to the condition (a3), inorganic spherical particles need to be dispersed in the first polymerization curable composition (A) such that the above-mentioned radial distribution function graph has a short-range order satisfying the following conditions (I) and (II):

(I) a closest particle-to-particle distance $r_1$ defined as r corresponding to a peak top of a peak closest to an origin among peaks appearing in the radial distribution function graph is a value being 1 to 2 times the average particle diameter $r_0$ of all the inorganic spherical particles dispersed in the cured product; and
(II) when r corresponding to a peak top of a peak second closest to the origin among the peaks appearing in the radial distribution function graph is represented as a second closest particle-to-particle distance $r_2$, a local minimum value of the radial distribution function g(r) between the closest particle-to-particle distance $r_1$ and the second closest particle-to-particle distance $r_2$ is a value of 0.56 to 1.10.

When inorganic spherical particles are dispersed in the first polymerization curable composition (A) such that the above-mentioned conditions (I) and (II) are satisfied, in the cured product (A') obtained by curing the first polymerization curable composition (A), the incident light is subjected to diffractive interference according to Bragg conditions, and light of a specific wavelength is emphasized without being affected by the incident angle of light, structural color corresponding to the average primary particle diameter is produced, and as a result, a structural color of a constant color tone can be produced.

In the first polymerization curable composition (A), as a method of quantifying the dispersed state of the inorganic spherical particles, the "radial distribution function g(r) within a plane" disclosed in Patent Document 3 is used, and thus the short-range order structure is defined. Here, as found from the use of the radial distribution function g(r) in Patent Document 3, the radial distribution function g(r) is well known as a function for determining a probability that a particle is present at a distance of r away from a given particle, and is defined by the above-mentioned formula (1). In formula (1) described above, $<\rho>$ represents the average particle density of particles within the plane, dn represents the number of particles which are present in a region between a circle having a radius r and a circle having a radius r+dr respectively with any one of the particles within the plane being the center and da represents $2\pi r \cdot dr$, which is the area of the region described above.

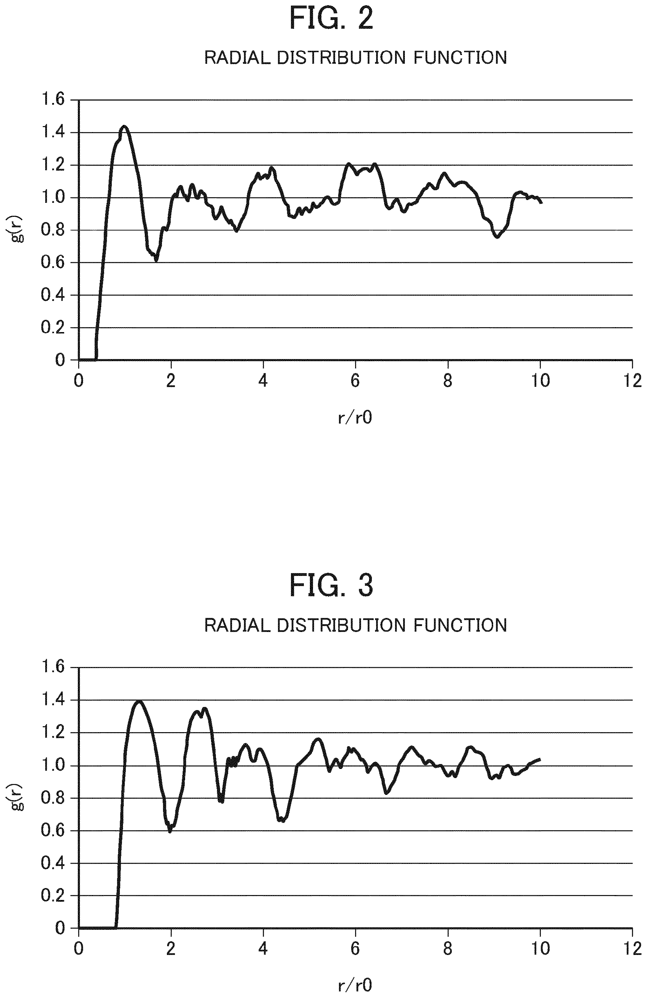

In general, the radial distribution function g(r) is represented by a radial distribution function graph in which a distance r is taken along an x-axis (distance axis) and in which the value of g(r) at r (the result of a calculation by formula (1) described above) is taken along a y-axis (vertical axis) or a radial distribution function graph (see Figs. 2 to 6) in which a dimensionless number standardized by dividing r by the average particle diameter of the particles is taken along a distance axis and in which the value of g(r) at r corresponding to the value of the x-axis (the result of a calculation by the formula described above) is taken along the y-axis (vertical axis).

In the present embodiment, since $<\rho>$ and dn are confirmed easily and reliably, based on $<\rho>$, dn and da (= $2\pi r \cdot dr$) corresponding to the value of dr employed when dn described above is determined, which are determined based on a scanning electron microscope image in which a plane within the cured product (A') obtained by curing the first polymerization curable composition (A) is used as an observation plane, g(r) calculated by formula (1) described above is employed.

The determination of $<\rho>$, dn and da can be performed as follows. Firstly, by a means such as polishing of the surface of the cured product (A') obtained by curing the first polymerization curable composition (A), the plane (observation plane) on which the dispersed state of the inorganic spherical particles in the cured product (A') can be observed is exposed on the surface. Then, the observation plane is observed with a scanning electron microscope, and thus a microscope image of a region in which at least 500 or more inorganic spherical particles are contained within the plane is acquired. Thereafter, for the obtained scanning electron microscope image, with image analysis software (for example, "Simple Digitizer ver. 3.2" free software), coordinates of the inorganic spherical particles within the region are determined. The coordinates of any one of inorganic spherical particles are selected from obtained coordinate data, with the selected inorganic spherical particle being the center, a circle in which at least 200 or more inorganic spherical particles are included and in which a distance r is the radius is drawn, and the number of inorganic spherical particles included within the circle is counted, with the result that the average particle density $<\rho>$ (unit: pieces/cm$^2$) can be determined.

With respect to dn, dr, having a length of a value of about $r_0/100$ to $r_0/10$ when the average particle diameter of the inorganic spherical particles is represented as $r_0$, is set, one inorganic spherical particle arbitrarily selected is assumed to be a central particle, the number of inorganic spherical particles included within a region between a circle in which a distance r from the center thereof is the radius and a circle which has the same center as the circle described above and which has a radius r+dr is counted, and thus dn can be determined. Furthermore, da, which is the area of the region between the two circles, is determined as $2\pi r \cdot dr$ based on the length of dr, which is actually set.

In the first polymerization curable composition (A), when the dimensionless number ($r/r_0$) standardized by dividing the distance r from the center of any one of the inorganic spherical particles dispersed in the cured product (A') by the average particle diameter $r_0$ of all the inorganic spherical particles dispersed in the cured product (A') is plotted on the x-axis, and the radial distribution function g(r) indicating the probability of finding an inorganic spherical particle at a distance of r away from the center of a given inorganic spherical particle is plotted on the y-axis and a radial distribution function graph indicating the relationship of $r/r_0$ and g(r) which corresponds to r at that time is created, the closest particle-to-particle distance $r_1$ which is defined as r corresponding to the peak top of the peak closest to the origin among the peaks appearing in the radial distribution function graph needs to be the value that is 1 to 2 times the average particle diameter $r_0$ of all the inorganic spherical particles dispersed in the cured product (A') (Condition I). When $r_1$ is less than one times $r_0$ ($r_1/r_0 < 1$), the amount of overlap of the particles within the plane is increased whereas when $r_1$ is greater than two times $r_0$ ($r_1/r_0 > 2$), particles are not present in the vicinity of the selected inorganic particle in the center, and thus the short-range order disappears, with the result that a structural color is prevented from being developed. In other words, from the viewpoint that the short-range order is maintained and a structural color is easily developed, $r_1/r_0$ is 1.0 to 2.0 and is preferably 1.0 to 1.5.

In the first polymerization curable composition (A), when r corresponding to the peak top of the peak second closest to the origin among the peaks appearing in the radial distribution function graph is represented as a second closest particle-to-particle distance $r_2$, the local minimum value of the radial distribution function g(r) between the closest particle-to-particle distance $r_1$ and the second closest particle-to-particle distance $r_2$ also needs to be a value of 0.56 to 1.10 (Condition II). When the local minimum value described above is less than 0.56, the long-range order of the arrangement structure of the inorganic spherical particles is increased, and thus not only the incident angle dependence of light of a structural color which is developed is increased, but also the chroma of the cured product is increased, with the result that it is difficult to obtain color tone compatibility when the cured product is used as a dental filling material. On the other hand, when the local minimum value described above exceeds 1.10, the arrangement structure of the inorganic spherical particles is a random structure, and thus it is difficult to obtain intended reflection performance, with the result that a desired structural color is unlikely to be developed. In other words, from the viewpoint that a structural color is developed, and the color tone compatibility of the composite material serving as a dental filling material is easily obtained, the local minimum value described above is a value of 0.56 to 1.10 and is preferably a value of 0.56 to 1.00.

The satisfaction of condition (I) means that the inorganic spherical particles are dispersed while holding constant short-range order, and the satisfaction of condition (II) means that the inorganic spherical particles are dispersed in a state where long-range order is random while short-range order is being maintained (which is not a completely random state where short-range order collapses and which is a state where fine domains having short-range order are randomly dispersed).

In order to easily satisfy these conditions, when the first polymerization curable composition (A) is prepared, it is preferable that the organic-inorganic composite fillers mentioned below are used as inorganic spherical particles are mixed while the particle diameter of the particles is controlled such that they are in a range of 5 $\mu$m to 50 $\mu$m, and particularly in a range of 5 $\mu$m to 30 $\mu$m, or coagulated particles of primary particles are mixed such that they are controlled such that a particle diameter is in a range from 5 $\mu$m to 200 $\mu$m and preferably a particle diameter in a range from 10 $\mu$m to 100 $\mu$m. When air bubbles are mixed during the mixing, since not only it is difficult to satisfy the conditions described above, but also a defect in the cured product is caused. It is thus preferable to perform, for example, defoaming treatment such that air bubbles are not left at least after the mixing. With attention being given to such points, sufficient mixing is performed using, for example, a mechanical mixer so as not to cause insufficient mixing, the above conditions can be surely satisfied. Note here that the inventors of the present invention have determined that when a curable composition (CR) is prepared by a manual kneading operation in accordance with Patent Document 4, even at a very low frequency, it is not possible to obtain a composition that exhibits the desired effect, and that when the radial distribution function g(r) is evaluated for such a system (in which the effect is not obtained), the above condition II (see Reference Comparative Example 2) is not satisfied.

[1-4. Preferred embodiment in curable composition first polymerization curable composition (A)]

In the first polymerization curable composition (A), since the short-range order structure described above can be obtained more simply and reliably, it is preferable that at least part of one or more identical particle diameter spherical particle groups include one type of identical particle diameter spherical particle group and a resin having a refractive index that is less than the refractive index of the inorganic spherical particles of the one type of identical particle diameter spherical particle group, and are blended as an organic-inorganic composite filler (that is, an organic-inorganic composite filler including only a single G-PID) which does not include an identical particle diameter spherical particle group other than the one type of the identical particle diameter spherical particle group.

Here, the organic-inorganic composite filler means a filler made of a powder formed of a composite in which an inorganic filler is dispersed in an (organic) resin matrix or a coagulated material in which the primary particles of an inorganic filler are bonded together with an (organic) resin.

In the preferable embodiment described above, for example, when three types of G-IDP having different average primary particle diameters, that is, G-PID$_1$, G-PID$_2$ and G-PID$_3$ are included, all or part of at least one type thereof is mixed as the "organic-inorganic composite filler including only a single G-PID". If all of G-PID$_1$ is blended as the organic-inorganic composite filler including only G-PID$_1$ (composite filler 1) in the first polymerization curable composition (A), within the composite filler 1, only G-PID$_1$ is included, and thus a short-range order structure for developing the structural color of G-PID$_1$ is realized, with the result that even in the cured product (A') obtained by curing the first polymerization curable composition (A), the structural color of G-PID$_1$ is reliably developed. When G-PID$_1$ is blended without being formed into a composite filler, since G-PID$_1$ is kneaded with G-PID$_2$ and G-PID$_3$ which are blended simultaneously (without being formed into a composite), it is thought that the constituent particles of G-PID$_1$ and the constituent particles of G-PID$_3$ are somewhat substituted with each other, that thus the closest particles of the inorganic spherical particles of G-PID$_1$ are formed into the inorganic particles of G-PID$_3$ and that in a region where the inorganic spherical particles are the center, the short-range order structure is destroyed. On the contrast, when all of G-PID$_1$ is blended as the composite filler 1, the mutual substitution of the particles as described above does not occur, the short-range order structure is not destroyed, and thus it is possible to minimize the proportion of the inorganic spherical particles which do not involve the development of the structural color, with the result that even in the cured product (A'), the structural color of G-PID$_1$ can be reliably developed. Likewise, G-PID$_1$ and/or G-PID$_3$ are blended as an organic-inorganic composite filler including only G-PID$_2$ (composite filler 2) and/or an organic-inorganic composite filler including only G-PID$_3$ (composite filler 3), and thus the structural colors thereof can be reliably developed.

From the viewpoint that the effect described above can be expected and furthermore, the viscosity of the first polymerization curable composition (A) is easily adjusted, 10% to 90%, preferably 20% to 80%, and more preferably 30% to 70% of each G-PID is blended as the "organic-inorganic composite filler including only a single G-PID".

When G-PID is blended in a form other than the form of "organic-inorganic composite filler including only a single G-PID", G-PID is generally blended in the form of a powder (G-PID itself serving as the aggregate of the inorganic spherical particles), but G-PID can be blended as an organic-inorganic composite filler including a plurality of types of G-PID. A more detailed description of the organic-inorganic composite filler including this case will be given below.

<Organic-inorganic composite filler>

As described above, the organic-inorganic composite filler means a filler made of a powder including a complex in which inorganic fillers are dispersed in an (organic) resin matrix or a coagulated material in which the primary particles of an inorganic filler are bonded together with an (organic) resin.

In the first polymerization curable composition (A), the inorganic spherical particles are used as the inorganic filler, and the resin having a refractive index that is less than that of the inorganic spherical particles is used as the resin forming the (organic) resin matrix. The resin described above is not particularly limited as long as the resin satisfies the condition as described above, but the resin is preferably a cured product of the first polymerizable monomer component used in producing the resin matrix of the cured product (A'). Although at this time, the resin does not need to have exactly the same composition as that of the first polymerizable monomer component, the resin having a refractive index being substantially equal to the refractive index of the polymerizable monomer is preferably used. When the refractive index of the resin described above is represented as $n_{(R)}$, and the refractive index of the inorganic spherical particles is represented as $n_{(F)}$, for any organic-inorganic composite filler, the following relationship:

$$n_{(R)} < n_{(F)}$$

needs to hold true. When the organic-inorganic composite filler includes inorganic spherical particles having a different refractive index, the relationship described above needs to hold true for all the inorganic spherical particles. $\Delta n$ (= $n_{(F)}$ - $n_{(R)}$), which is a difference between $n_{(F)}$ and $n_{(R)}$, is preferably 0.001 to 0.01, and more preferably 0.001 to 0.005.

The content of inorganic spherical particles in the organic-inorganic composite filler is preferably 30% by mass to 95% by mass. When the content to the organic-inorganic composite filler is 30% by mass or more, the colored light of the cured product (A') of the first polymerization curable composition (A) is satisfactorily developed, and mechanical strength can also be sufficiently enhanced. It is difficult to perform an operation of containing more than 95% by mass of the inorganic spherical particles in the organic-inorganic composite filler, and it is difficult to obtain a homogeneous mixture. The more suitable amount of the inorganic spherical particles mixed in the organic-inorganic composite filler is 40% by mass to 90% by mass.

The organic-inorganic composite filler can be produced according to a general production method in which predetermined amounts of individual components of the inorganic spherical particles, the polymerizable monomer, and the polymerization initiator are mixed together, and in which the mixture is polymerized by a method such as heating or light application, followed by pulverization. With such a method, it is possible to obtain the irregularly shaped organic-inorganic composite filler formed of a composite in which the inorganic spherical particles are dispersed in the resin matrix.

The organic-inorganic composite filler can also be produced by a method disclosed in PCT International Publication Nos. WO2011/115007 and WO2013/039169, that is, a method of immersing coagulated particles formed of a coagulated material of inorganic spherical particles in a liquid composition including a polymerizable monomer, a polymerization initiator and an organic solvent, removing the organic solvent, and polymerizing and curing the polymerizable monomer by a method such as heating or light application. With such a method, while a state where the primary particles of the inorganic spherical particles are coagulated is being substantially kept, a resin covers at least a part of the surfaces of each of the primary particles, the primary particles are bonded to each other, and thus it is possible to obtain a porous organic-inorganic composite filler having a large number of fine holes which communicate with the outside.

The average particle diameter of the organic-inorganic composite filler is preferably 2 μm to 100 μm, more preferably 5 μm to 50 μm, and further preferably 5 μm to 30 μm from the viewpoint that the conditions (I) and (II) are easily satisfied and further the mechanical strength of a curable body and the operability of the curable paste are improved.

A pigment, a polymerization inhibitor, a fluorescent brightening agent, and the like, can be added to the organic-inorganic composite filler as long as the effects thereof are not inhibited (in general, 0.0001 parts by mass to 5 parts by mass thereof with respect to 100 parts by mass of the organic-inorganic composite filler is added). The organic-inorganic composite filler may be surface-treated with a silane coupling agent or the like.

The amount of organic-inorganic composite filler is preferably determined, with consideration given to the amount of identical particle diameter spherical particle group not being formed into the organic-inorganic composite filler blended in the first polymerization curable composition (A), by conversion from the amount of inorganic spherical particles included in the organic-inorganic composite filler such that the total amount of G-PID (i.e., the total amount of inorganic spherical particles) falls into the range described above.

[1-5. Other additives in first polymerization curable composition (A)]

Other additives such as a polymerization inhibitor and a UV absorber can be blended in the first polymerization curable composition (A) as long as the effects thereof are not inhibited.

The cured product (A') obtained from the first polymerization curable composition (A) develops, as described above, a structural color without use of a colorant component (coloring substance) such as a pigment and a dye substance. Hence, a colorant component which is likely to discolor as time passes does not need to be mixed in the first curable composition (A). However, the blending of a colorant component itself is not completely denied, and a colorant may be blended as long as the colorant does not prevent colored light caused by interference of a spherical filler. Specifically, about 0.0005 to 0.5 parts by mass, and preferably, about 0.001 to 0.3 parts by mass of a colorant component with respect to 100 parts by mass of the first polymerizable monomer component may be mixed.

[2. Second polymerization curable composition (B)]

A second polymerization curable composition (B) is a polymerization curable composition for forming a foundation layer serving as a foundation of a surface exposed layer including the cured product (A') of the first polymerization curable composition (A). In the second polymerization curable composition (B), since the cured product (B') has predetermined value, the presence of the cured product (B') as a foundation of the surface exposed layer reduces the amount of light passing through the surface-exposed layer to an appropriate extent similar to dentin existing deep in the cavities, so that good color tone compatibility can be exhibited even when the cured body (B') is used for the restoration of cavities of class III and class IV.

[2-1. Conditions (b1) and (b2)]

A second polymerization curable composition (B) contains a second polymerizable monomer component, a colorant component, and a second polymerization initiator component (condition (b1)). The colorant component is blended such that the value (V) of the colorimetric value measured by the Munsell color system of the colored light when measured under a black background using a color difference meter on a 1 mm-thick sample including the cured product (B') of the second polymerization curable composition (B) is 5 or more (condition (b2)).

As the second polymerizable monomer component and the second polymerization initiator component, components conventionally used for dental filling restorative material can be used without any particular limitation, and components being examples of the first polymerizable monomer component and the polymerization initiator component can be suitably used. Furthermore, the second polymerization curable composition (B) may contain a filling material. However, there is no limitation as to the refractive index between the filling material and the second polymerizable monomer component (more specifically, the cured product thereof). In the second polymerization curable composition (B), a (meta)acrylic polymerizable monomer is preferably used as the second polymerizable monomer component, and preferably, 50 parts by mass to 1500 parts by mass of filling materials, and an effective amount of polymerization initiator are included with respect to 100 parts by mass of the (meta)acrylic polymerizable monomer.

Furthermore, as the colorant component (also referred to as a coloring substance or coloring material), a pigment and/or a dye substance is used. When the colorant component is a pigment, an inorganic pigment is suitably used. Examples of inorganic pigments to be suitably used include titanium oxide, zinc oxide, zirconium oxide, zinc sulfide, aluminum silicate, calcium silicate, carbon black, iron oxide, copper chromite black, chromium oxide green, chromium green, violet, chromium yellow, lead chromate, lead molybdate, cadmium titanate, nickel titanium yellow, ultramarine blue, cobalt blue, bismuth vanadate, cadmium yellow, cadmium red, and the like. Note here that in this specification, the inorganic pigment also corresponds to an inorganic filler material. Furthermore, organic pigments such as monoazo pigments, diazo pigments, diazo condensation pigments, perylene pigments, anthraquinone pigments, and the like, can also be used. Furthermore, when the colorant component is a dye substance, as the dye substance, reddish dye substance such as KAYASET RED G, KAYASET RED B (both are manufactured by Nippon Kayaku Co., Ltd.); yellowish dye substance such as KAYASET Yellow 2G, KAYASET Yellow GN (both are manufactured by Nippon Kayaku Co., Ltd.); bluish dye substance such as KAYASET Blue N, KAYASET Blue G, KAYASET Blue B (all are manufactured by Nippon Kayaku Co., Ltd.); and the like, can be used. In view of the color tone stability within an oral cavity, water-insoluble pigment is more preferably used than water-soluble dye substance.

Also in conventionally common dental filling restorative materials, a colorant component is mixed in order to match the color tone of teeth, gingiva, and crown material. However, in the second polymerization curable composition (B), in order to form a foundation layer having the above-mentioned effect, the cured product (B') needs predetermined value.

Herein, the desired value means that the value (V) of the colorimetric value of the colored light measured by the Munsell color system when measured with a color difference meter under a black background for a 1 mm-thick sample made of the cured product (B') of the second polymerization-curable composition (B) is 5 or more. The above-mentioned value (V) can be obtained by measuring a spectral reflectivity in a black background using the color-difference meter (for example, "TC-1800MKII" manufactured by Tokyo Denshoku Co., Ltd., and the like), under black background (foundation having value of 1 by the Munsell color system).

When the value (V) is less than 5, a restored product tends to be dark and less aesthetic. The value (V) is preferably 5.5 or more, and more preferably 6 or more. Furthermore, when the value is too high, the reflection of light becomes large, and the visibility of the structural color developed by the surface restored product is lowered, and the harmony between the restored portion and the periphery of the teeth to be restored may be lost. Therefore, the value is preferably 9 or less, more preferably 8.5 or less, further preferably 8 or less, and particularly preferably 7.5 or less.

The value can be controlled by appropriately adjusting the blending amount according to types of the colorant component to be used. Specifically, in order to enhance the value (V), the blending amount of the white pigment is increased, and the blending amount of the red, yellow, blue, and the like, particularly the blending amount of the blue, may be decreased.

[3. Configuration of dental filling restorative material kit]

The dental filling restorative material kit according to the present embodiment may include only the first polymerization curable composition (A) and the second polymerization curable composition (B), but may further include a polymerization curable composition having a special color tone for forming an intermediate layer in order to better match the color tone of the human tooth to be restored with the restorative material. Specifically, composition may further include a polymerization-curable composition of a white color having high value (value greater than 7) suitable for opaque white teeth after bleaching treatment, a polymerization-curable composition of a transparent color having value of less than 4 suitable for teeth with high transparence, and the like.

The polymerization curable composition having such a color tone is adopted from general dental filling restorative material, and a composite resin containing a polymerizable monomer, a polymerization initiator, a filling material, and a coloring material is used.

[4. Method for using dental filling restorative material kit]

A dental filling restorative material kit according to the present embodiment can be suitably used for restoring a cavity in which no dentin is present in the deep portion, i.e., a cavity of class III which is adjacent to the anterior tooth and which does not include incisal angle, and a cavity of class IV which is a cavity adjacent to the anterior tooth and which includes the incisal angle.

When these cavities are restored, firstly, the second polymerization-curable composition (B) is disposed in a deep portion of the cavity (lost portion) or behind the cavity to form a foundation layer by adjusting the thickness (usually, 0.1 mm to 2 mm) and a shape thereof, and after curing as necessary, the first polymerization-curable composition (A) is disposed on the uncured foundation layer or cured foundation layer to adjust the shape thereof (together with the foundation layer if the foundation layer is uncured), and after curing, a polishing process is performed.

EXAMPLES

The present invention will be more specifically described below with reference to Examples, but the present invention is not limited to these Examples.

Firstly, it was demonstrated that a first polymerization curable composition (A) (structural color development curable composition) gives a cured product developing the desired structural color, in other words, that the cured product (A') develops the desired structural color when conditions (a1), (a2), and (a3) are satisfied, based on the following Reference Examples and Reference Comparative Examples (corresponding to Examples and Comparative Examples of Patent Document 5).

[Reference Example and Reference Comparative Example]

1. Polymerizable monomer component

As polymerizable monomer mixtures having the compositions shown in Table 1, M1 and M2, were used. Symbols in the polymerizable monomer column of the Table represent compounds below respectively, and numbers within parentheses following abbreviation indicate used parts by mass.

- UDMA: 1,6-bis (methacrylethyloxycarbonylamino) trimethylhexane
- 3G: triethylene glycol dimethacrylate
- bis-GMA: 2,2-bis[(3-methacryloyloxy-2-hydroxypropyloxy)phenyl]propane

The viscosities of M1 and M2 were measured with an E-type viscometer ("VISCONIC ELD" manufactured by Tokyo Keiki Co., Ltd.) in a constant temperature chamber of 25°C.

The refractive index before curing (M1 or M2) and the refractive index after curing (cured product) were measured using an Abbe refractometer (manufactured by Atago Co., Ltd.) in the constant temperature chamber of 25°C. At this time, the cured product sample was produced as follows: 0.2% by mass of camphorquinone (CQ), 0.3% by mass of ethyl p-N,N-dimethylaminobenzoate (DMBE), and 0.15% by mass of hydroquinone monomethyl ether (HQME) were added to 100 parts by mass each of M1 or M2 as the polymerization initiator, and were uniformly mixed, the resulting mixture was put into a mold having a through-hole of 7 mm$\phi \times$ 0.5 mm, polyester films were pressed on both sides, followed by curing thereof by light application for 30 seconds with a halogen-type dental light irradiator ("Demetron LC" manufactured by Sybron Dental Specialties Inc.) having a light intensity of 500 mW/cm$^2$, and thereafter the cured product was taken out of the mold. When the cured product sample was set in the Abbe refractometer, in order for the cured product sample and a measurement surface to be brought into intimate contact with each other, a solvent (bromonaphthalene) not dissolving the sample and having a higher refractive index than the sample was dropped onto the sample.

[Table 1]

| Monomer name | Polymerizable monomer | Viscosity of polymerizable monomer [mPa ∙ s] | Refractive index | |
|---|---|---|---|---|
| | | | Before curing | After curing |
| M1 | UDMA(60)/3G(40) | 150.14 | 1.474 | 1.509 |
| M2 | bis-GMA (60) /3G (40) | 755.65 | 1.515 | 1.546 |

2. Inorganic particles

2-1. Identical particle diameter spherical particle group (G-PID)

As G-PID, G-PID1 to G-PID11 shown in Table 2 were used. These identical particle diameter spherical particle groups were prepared according to a method (so-called sol-gel method) disclosed in Japanese Unexamined Patent Application Publications Nos. 58-110414 and 58-156524 and the like. Specifically, a mixture solution which included a hydrolyzable organosilicon compound (such as tetraethyl silicate) and a hydrolyzable organic titanium group metal compound (such as tetrabutylzirconate or tetrabutyltitanate) such that compositions shown in the composition column of Table 2 were provided was added into an ammonia alcohol solution (for example, methanol, ethanol, isopropyl alcohol or isobutyl alcohol) into which ammonia water was introduced and hydrolysis was performed to precipitate a reaction product. Then, the resulting precipitate was separated, thereafter dried, and calcined after being pulverized as necessary to obtain the calcined product.

Then, 4 parts by mass of γ-methacryloyloxypropyltrimethoxysilane and 3 parts by mass of n-propylamine with respect to 100 parts by mass of the obtained calcined product were stirred and mixed in 500 parts by mass of methylene chloride, methylene chloride was removed with an evaporator and thereafter heat-drying was performed at 90°C, with the result that surface-treated identical particle diameter spherical particle groups were obtained.

Note here that the average particle diameter in Table 2 (which means an average primary particle diameter for inorganic spherical particles), the rate of particles within ±5% [which means the rate (%) of the number of particles being present within a range of plus or minus 5% of the average primary particle diameter in the number-based particle size distribution with respect to the total number of particles], the average uniformity, and the refractive index were measure as follows.

(1) Average primary particle diameter

A photograph of a powder was shot with a scanning electron microscope ("XL-30S", manufactured by Philips N.V.) at a magnification of 5000 to 100000 times, the shot image was processed with image analysis software ("IP-1000PC" manufactured by Asahi Kasei Engineering Corp.), the number (30 or more particles) and the primary particle diameter (maximum diameter) of particles observed within a unit field of view of the photograph were measured and the number average primary particle diameter was calculated by a formula below based on the measured values.

$$\bar{x} = \frac{\sum_{i=1}^{n} x_i}{n} \text{ (Number average)}$$

**(n: number of particles, $x_i$: primary particle diameter (maximum diameter) of i-th particle)** (2) Rate of particles within range of ±5% [rate (%) of the number of particles being present within a range of plus or minus 5% of the average primary particle diameter in the number-based particle size distribution with respect to the total number of particles]

Among all particles (30 or more particles) within a unit field of view of the photograph described above, the number of particles having a primary particle diameter (maximum diameter) outside a particle diameter range of plus or minus 5% of the average primary particle diameter determined above was measured, the measured value was subtracted from the number of all particles, the number of particles within the particle diameter range of plus or minus 5% of the average primary particle diameter within the unit field of view of the photograph was determined, calculation was performed according to a formula below:

Rate of particles within range of ±5% = [(number of particles within particle diameter range of plus or minus 5% of average primary particle diameter within unit field of view of scanning electron microscope photograph)/(number of all particles within unit field of view of scanning electron microscope photograph)] × 100

(3) Average uniformity

A photograph of the powder was shot with the scanning electron microscope, the number (n: 30 or more) of particles of the identical particle diameter spherical particle group (G-PID) observed within a unit field of view of the photograph, the major diameter (Li), which was the maximum diameter of the particle, and the minor diameter (Bi), which was a diameter in a direction orthogonal to the major diameter, were determined, and thus the average uniformity was calculated by a formula below.

$$\text{Average uniformity} = \frac{\sum_{i=1}^{n} Bi/Li}{n}$$

(4) Refractive index

Measurement was performed by a liquid immersion method using the Abbe refractometer (manufactured by Atago Co., Ltd.). Specifically, in the constant temperature chamber of 25°C, within a sample bottle of 100 mL, the identical particle diameter spherical particle group (G-PID) was dispersed in 50 mL of anhydrous toluene. While this dispersion liquid was being stirred with a stirrer, 1-bromotoluene was dropped little by little, the refractive index of the dispersion liquid when the dispersion liquid was the most transparent was measured, and the obtained value was used as the refractive index of the identical particle diameter spherical particle group (G-PID).

[Table 2]

| | Composition, shape of filler | | Average primary particle diameter (nm) | Refractive index | Ratio of particles within ±5% (%) | Average uniformity |
|---|---|---|---|---|---|---|
| | Composition (mol%) SiO$_2$ / ZrO$_2$ / Na$_2$O | Shape | | | | |
| G-PID1 | 89.8 / 9.0 / 1.2 | spherical | 80 | 1.515 | 91 | 0.98 |
| G-PID2 | 89.8 / 9.0 / 1.2 | spherical | 200 | 1.515 | 93 | 0.97 |
| G-PID3 | 89.8 / 9.0 / 1.2 | spherical | 238 | 1.515 | 96 | 0.95 |
| G-PID4 | 89.8 / 9.0 / 1.2 | spherical | 250 | 1.515 | 95 | 0.92 |
| G-PID5 | 89.8 / 9.0 / 1.2 | spherical | 262 | 1.515 | 95 | 0.94 |
| G-PIDG | 89.8 / 9.0 / 1.2 | spherical | 275 | 1.515 | 92 | 0.93 |
| G-PID7 | 89.8 / 9.0 / 1.2 | spherical | 280 | 1. 515 | 94 | 0.93 |
| G-PID8 | 89.8 / 9.0 / 1.2 | spherical | 295 | 1.515 | 92 | 0.94 |
| G-PID9 | 89.8 / 9.0 / 1.2 | spherical | 331 | 1.3215 | 92 | 0.92 |
| G-PID10 | 89.8 / 9.0 / 1.2 | spherical | 367 | 1.515 | 90 | 0.94 |
| G-PID11 | 89.8 / 9.0 / 1.2 | spherical | 400 | 1. 515 | 91 | 0.94 |
| F1 | 89.8 / 9.0 / 1.2 | irregular shape | 500 | 1.515 | 50 | - |

2-2. Organic-inorganic composite filler (CF1)

To 200 g of water, 100 g of the identical particle diameter spherical particle group (G-PID5) shown in Table 2 was added to obtain a water dispersion liquid thereof using a circulation-type pulverizer SC mill (manufactured by Nippon Coke & Engineering Co., Ltd.).

On the other hand, 4 g (0.016 mol) of γ-methacryloyloxypropyltrimethoxysilane and 0.003 g of acetic acid were added to 80 g of water, and the obtained product was stirred for 1 hour and 30 minutes to obtain a uniform solution of pH 4. This solution was added to the water dispersion liquid described above, and mixed until the dispersion liquid became uniform. Thereafter, while the dispersion liquid was being mixed lightly, the dispersion liquid was supplied onto a disc rotating at high speed and was granulated by a spray drying method. Spray drying was performed using a spray dryer TSR-2W (manufactured by Sakamoto Giken Co., Ltd.) provided with a rotating disc and sprayed by centrifugal force. The rotation speed of the disc was 10000 rpm, and the temperature of air in a dry atmosphere was 200°C. Thereafter, a powder obtained by being granulated by spray drying was dried in a vacuum at 60°C for 18 hours, and 73 g of a substantially spherical coagulated material was obtained.

Then, 50 g of the coagulated material described above was immersed in a polymerizable monomer solution (containing 36 parts by mass of the polymerizable monomer component with respect to 100 parts by mass of an organic solvent) obtained by mixing 10 g of the polymerizable monomer component M1, 0.025 g of azobisisobutyronitrile (AIBN) serving as a thermal polymerization initiator and furthermore 5.0 g of methanol serving as the organic solvent. The resulting mixture was sufficiently stirred, followed by checking that the mixture was in a slurry state and the slurry was left to stand for one hour.

The mixture described above was transferred into a rotary evaporator. In a stirred state, the mixture was dried for one hour under conditions in which the degree of pressure reduction was 10 hPa and in which a heating condition was 40°C (a warm water bath was used), with the result that the organic solvent was removed. When the organic solvent was removed, a powder having high fluidity was obtained. While the obtained powder was being stirred in the rotary evaporator, the powder was heated for one hour under conditions in which the degree of pressure reduction was 10 hPa and in which a heating condition was 100°C (an oil bath was used), and thus the polymerizable monomer in the powder was polymerized and cured. With this operation, 45 g of the substantially spherical organic-inorganic composite filler (CF1) in which the surface of a spherical coagulated material was coated with an organic polymer was obtained. The average particle diameter of the organic-inorganic composite filler was 33 μm.

2-3. Superfine particles (G-SFP)

As G-SFP, REOLOSIL QS102 (average primary particle diameter of 30 nm, manufactured by Tokuyama Corporation) was used.

2-4. Irregularly shaped inorganic particles

Irregularly shaped inorganic particles F1 shown in Table 2 were used. The irregularly shaped inorganic particles F1 were prepared according to a method disclosed in Japanese Unexamined Patent Application Publications Nos. 2-132102 and 3-197311 and the like as follows: an alkoxysilane compound was dissolved in an organic solvent, water was added thereto so as to perform partial hydrolysis, thereafter an alkoxide of another metal and an alkali metal compound which were formed into a composite were further added so as to perform hydrolysis and to thereby generate a gel-like material and then the gel-like material was dried, was thereafter pulverized as necessary, and was calcined. The average primary particle diameter (which means, for the irregularly shaped particles, the average particle diameter of pulverized particles), the rate of particles within the range of plus or minus 5% and the refractive index were measured as in G-PID.

3. Polymerization initiator

As the polymerization initiator, a polymerization initiator formed by combination of camphorquinone (CQ), ethyl p-N,N-dimethylaminobenzoate (DMBE), and hydroquinone monomethyl ether (HQME) was used.

<Reference Example 1>

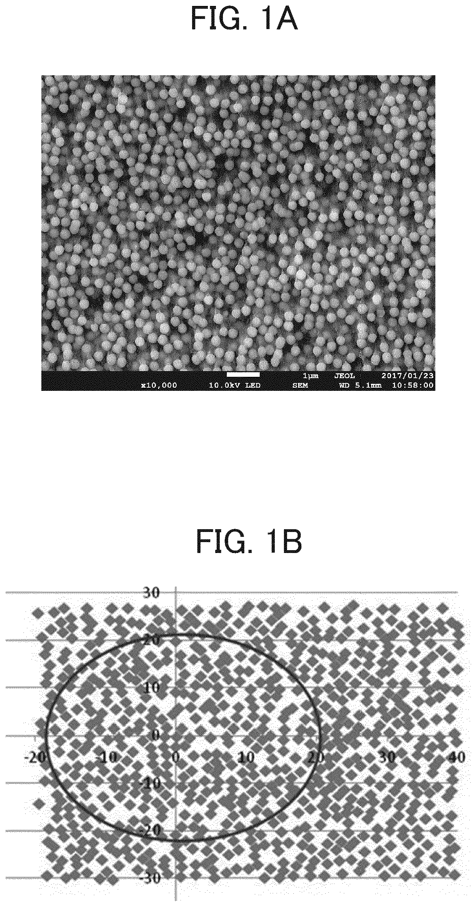

To 100 parts by mass of the polymerizable monomer component M1, 0.3 parts by mass of CQ, 1.0 part by mass of DMBE and 0.15 parts by mass of HQME were added, mixed together, and thus a uniform polymerizable monomer composition was prepared. Then, 400 parts by mass of G-PID4 and 0.5 parts by mass of a superfine particle group (G-SFP) were weighed, the polymerizable monomer composition described above was gradually added under red light and they were sufficiently kneaded with the use of a kneader planetary mixer (manufactured by INOUE MFG., INC.) to obtain a uniform curable paste. Furthermore, the paste was defoamed under reduced pressure, and thus air bubbles were removed, a polymerization curable composition {first polymerization curable composition (A)} was prepared. On the cured product (A') of the obtained polymerization curable composition, (1) the visual evaluation of colored light, (2) the measurement of the wavelength of the colored light, (3) the evaluation of color tone compatibility with a colorimeter, (4) the visual evaluation of color tone compatibility, and (5) the evaluation of the radial distribution function of inorganic spherical particles were performed. The composition (in a matrix column, a polymerizable monomer component providing a resin for forming a matrix is described) of the polymerization curable composition and the results of the evaluations are shown in Tables 3 to 5. An example of a scanning electron microscope image in an observation plane of the cured product of Reference Example 1 is shown in Fig. 1A, an example of coordinate data obtained from the scanning electron microscope image is shown in Fig. 1B, and a radial distribution function graph on g(r) calculated based on a parameter determined from the coordinate data is shown in Fig. 2. In Reference Example 1, uniform compositions providing a cured product that satisfied conditions I and II of the radial distribution function were obtained at a ratio of 10 out of 10 tests with high reducibility.
The evaluations and measurements described above were performed by methods which will be described below.

(1) Visual evaluation of colored light

The polymerization curable composition (paste) was put into a mold having a through-hole of 7 mmφ × 1 mm, and polyester films were pressed on both sides. Curing was performed by light application for 30 seconds on both sides with the visible light irradiator (Power Light, manufactured by Tokuyama Corporation), and thereafter an evaluation sample was produced by being taken out of the mold. The obtained evaluation sample was placed on the adhesive surface of an about 10 mm square black tape (carbon tape), and the color tone of colored light was visually checked.

(2) Wavelength of colored light

On an evaluation sample formed in the same manner as in (1) described above, a spectral reflectivity was measured in a black background and in a white background with a color difference meter ("TC-1800MKII", manufactured by Tokyo Denshoku Co., Ltd.), and the local maximum point of the reflectivity in the black background was assumed to be the wavelength of the colored light.

(3) Evaluation of color tone compatibility with colorimeter

A hard resin tooth in which a cavity of class I (diameter of 4 mm and a depth of 2 mm) was reproduced in the center portion of the occlusal surface of lower right number 6 was used, the polymerization curable composition (paste) was filled in a lost portion, curing and polishing were performed and thus simulated restoration was performed. The color tone compatibility after the simulated restoration was evaluated with a two-dimensional colorimeter ("RC-500", manufactured by PaPaLab Co., Ltd.). As the hard resin tooth, a hard resin tooth of high chroma (corresponding to A4) and a hard resin tooth of low chroma (corresponding to A1) which were in the class of A system (reddish brown) in the shade guide ("VITA Classical", manufactured by Vita Zahnfabrik H. Rauter GmbH & Co. KG) and a hard resin tooth of high chroma (corresponding to B4) and a hard resin tooth of low chroma (corresponding to B1) which were in the class of B system (reddish yellow) in the shade guide ("VITA Classical", manufactured by Vita Zahnfabrik H. Rauter GmbH & Co. KG), were used.

The hard resin tooth was set in the two-dimensional colorimeter, the hard resin tooth was shot, image analysis software ("RC Series Image Viewer", manufactured by PaPaLab Co., Ltd.) was used to process the shot image and a color difference ($\Delta E^*$ in CLELab) between the colorimetric values of the restored portion and the non-restored portion of the hard resin tooth was determined, with the result that the evaluation of the color tone compatibility was performed.

$$\Delta E^* = \{(\Delta L^*)^2 + (\Delta a^*)^2 + (\Delta b^*)^2\}^{1/2}$$

$$\Delta L^* = L1^* - L2^*$$

$$\Delta a^* = a1^* - a2^*$$

$$\Delta b^* = b1^* - b2^*$$

L1*: lightness index of restored portion of hard resin tooth, a1*, b1*: c chroma index of restored portion of hard resin tooth, L2*: lightness index of non-restored portion of hard resin tooth, a2*, b2*: chroma index of non-restored portion of hard resin tooth and $\Delta E^*$: color tone variation amount

(4) Visual evaluation of color tone compatibility

The simulated restoration was performed in the same manner as in (3), and color tone compatibility after the restoration was visually checked. The criteria are shown below.

-Criteria-

5: Color tone of restored product was indistinguishable from hard resin tooth.
4: Color tone of restored product had good compatibility with hard resin tooth.
3: Color tone of restored product was similar to hard resin tooth.
2: Color tone of restored product was similar to hard resin tooth but was not satisfactorily compatible therewith.
1: Color tone of restored product was not compatible with hard resin tooth.

(5) Evaluation of radial distribution function of inorganic spherical particles

The polymerization curable composition (paste) was put into a mold having a through-hole of 5 mm$\phi$ × 10 mm, and polyester films were pressed on both sides. Curing was performed by light application for 30 seconds on both sides with the visible light irradiator (Power Light, manufactured by Tokuyama Corporation), and thereafter the cured product of the polymerization curable composition (paste) was obtained by being taken out of the mold. Specifically, on the cured product, cross section milling of the cured product was performed using an ion milling device ("IM4000" manufactured by Hitachi, Ltd.) under conditions of 2kV and 20 minutes, and thus an observation plane was formed. For the observation plane, with the scanning electron microscope, a microscope image of a region which contained 1000 spherical particles within the plane was acquired, and the coordinates of the spherical particles within the region above were analyzed on the obtained scanning electron microscope image with the image analysis software ("Simple Digitizer ver. 3.2" free software). One set of coordinates of any one of the spherical particles were selected from obtained coordinate data, with the selected spherical particle being the center, a circle in which at least 200 or more spherical particles were included and in which a distance r was the radius was drawn and the number of spherical particles included within the circle was determined, with the result that the average particle density $<\rho>$ (unit: pieces/cm$^2$) was calculated. dr was a value of about $r_0/100$ to $r_0/10$ ($r_0$ indicating the average particle diameter of the spherical particles), and the number dn of particles included within a region between a circle of a distance r from the spherical particle in the center and a circle of a distance r+dr and the area da of the region were determined. The values of $<\rho>$, dn and da determined as described above were used, and thus the following formula (1) was calculated:

$$g(r) = \{1/<\rho>\} \times \{dn/da\} \quad (1),$$

and the radial distribution function g(r) was determined. Then, a graph indicating a relationship between the radial distribution function and $r/r_0$ (r represented an arbitrary distance from the center of the circle and $r_0$ represented the average particle diameter of the spherical particles) was produced. Then, on conditions I and II of the radial distribution function, evaluations were performed with "S" indicating the satisfaction of the condition and "N" indicating the dissatisfaction of the condition.

[Table 3]

| No. | | Resin matrix | Identical particle diameter spherical particle group | Superfine particle group | Refractive index difference* | Colored light visual evaluation | Colored light wavelength (nm) Black background | Colored light wavelength (nm) White background | Radial distribution function | |
|---|---|---|---|---|---|---|---|---|---|---|
| | | | | | | | | | Condition I | Condition II |
| Reference Example | 1 | M1 (100) | G-PID4(400) | G-SFP (0.5) | 0.006 | yellow | 607 | no local maximum | S | S |
| | 2 | M1 (100) | G-PID5(200) / G-PID8 (200) | G-SFP (1) | 0.006 | red | 695 | no local maximum | S | S |
| | 3 | M1 (100) | G-PID4 (200) / G-PID7 (100) / G-PID9 (100) / G-PID10 (100) / G-PID11 (100) | G-SFP (1) | 0.006 | red | 756 | no local maximum | S | S |
| | 4 | M1 (100) 100) | CF1 (300) / G-PID5 | G-SFP (1) | 0.006 | red | 720 | no local maximum | S | S |
| Reference Comparative Example | 1 | M2 (100) | G-PID4 (400) | G-SFP (0.5) | -0.031 | blue | 481 | no local maximum | - | - |
| | 2 | M1 (100) | G-PID2 (400) | G-SFP (0.5) | 0.006 | dark blue | 430 | no local maximum | S | N |
| | 3 | M1 (100) | G-PID1 (400) | G-SFP (0.5) | 0.006 | none | 405 | no local maximum | - | - |
| | 4 | M1 (100) | F1 (400) | G-SFP (0.5) | 0.006 | none | no local maximum | no local maximum | - | - |
| | 5 | M1 (100) | G-PID3 (100) / G-PID4 (200) / G-PID5 (200) / G-PID6 (100) | G-SFP (1) | 0.006 | none | no local maximum | no local maximum | - | - |

*Refractive index of identical particle diameter spherical particle group (G-PID) - refractive index of polymer of resin matrix

[Table 4]

| No. | | A system (reddish blown) color tone compatibility | | | |
|---|---|---|---|---|---|
| | | Low chroma | | High chroma | |
| | | Visual evaluation | ΔE* | Visual evaluation | ΔE* |
| Reference Example | 1 | 4 | 0.82 | 4 | 0.85 |
| | 2 | 5 | 0.18 | 5 | 0.25 |
| | 3 | 5 | 0.20 | 4 | 0.85 |
| | 4 | 5 | 0.29 | 5 | 0.28 |
| Reference Comparative Example | 1 | 2 | 3.67 | 2 | 3.89 |
| | 2 | 1 | 8.53 | 1 | 8.37 |
| | 3 | 1 | 4.87 | 1 | 4.93 |
| | 4 | 2 | 3.99 | 1 | 4.71 |
| | 5 | 2 | 3.98 | 2 | 3.89 |

[Table 5]

| No. | | B system (reddish yellow) color tone compatibility | | | |
|---|---|---|---|---|---|
| | | Low chroma | | High chroma | |
| | | Visual evaluation | ΔE* | Visual evaluation | ΔE* |
| Reference Example | 1 | 5 | 0.32 | 5 | 0.20 |
| | 2 | 5 | 0.34 | 4 | 0.95 |
| | 3 | 5 | 0.33 | 4 | 0.88 |
| | 4 | 5 | 0.31 | 5 | 0.35 |
| Reference Comparative Example | 1 | 1 | 4.66 | 1 | 4.85 |
| | 2 | 1 | 7.86 | 1 | 7.94 |
| | 3 | 1 | 4.88 | 1 | 4.91 |
| | 4 | 2 | 3.98 | 1 | 4.81 |
| | 5 | 2 | 3.92 | 2 | 3.79 |

<Reference Example 2 to 4>

Cured products were obtained in the same manner as in Reference Example 1 except that the compositions of the polymerization curable composition were changed as shown in Table 3. On the obtained cured products, in the same manner as in Reference Example 1, (1) the visual evaluation of colored light, (2) the measurement of the wavelength of the colored light, (3) the evaluation of color tone compatibility with a colorimeter, (4) the visual evaluation of color tone compatibility and (5) the evaluation of the radial distribution function of inorganic spherical particles were performed. The results of the evaluations are shown in Tables 3 to 5. Radial distribution function graphs for the cured products of Reference Examples 2 to 4 are shown in Figs. 3 to 5. In Reference Examples 2 to 4, uniform compositions providing cured products satisfying the conditions I and II of the radial distribution function were obtained at a ratio of 10 out of 10 tests with high reducibility.

<Reference Comparative Examples 1, and 3 to 5>

Cured products were obtained in the same manner as in Reference Example 1 except that the compositions of the cured products were changed as shown in Table 3. On the obtained cured products, in the same manner as in Reference Example 1, (1) the visual evaluation of colored light, (2) the measurement of the wavelength of the colored light, (3) the evaluation of color tone compatibility with a colorimeter and (4) the visual evaluation of color tone compatibility were performed. The results of the evaluations are shown in Tables 3 to 5.

<Reference Comparative Example 2>

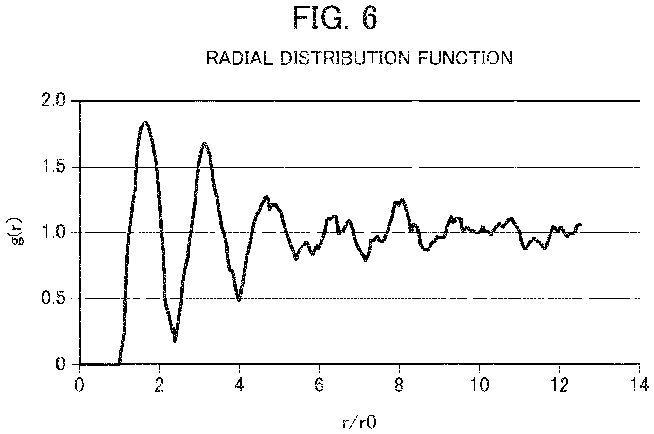

To 100 parts by mass of the polymerizable monomer component M1, 0.3 parts by mass of CQ, 1.0 part by mass of DMBE and 0.15 parts by mass of HQME were added, they were mixed together, and thus a uniform polymerizable monomer composition was prepared. Then, 400 parts by mass of G-PID2 and 0.5 parts by mass of the superfine particle group (G-SFP) were weighed, the polymerizable monomer composition described above was gradually added under red light, and they were kneaded with a mortar so as to form a curable paste. Furthermore, the paste was defoamed under reduced pressure, and thus air bubbles were removed, and polymerization curable composition was prepared. On the cured product of the obtained polymerization curable composition, (1) the visual evaluation of colored light, (2) the measurement of the wavelength of the colored light, (3) the evaluation of color tone compatibility with a colorimeter, (4) the visual evaluation of color tone compatibility and (5) the evaluation of the radial distribution function of inorganic spherical particles were performed. The composition (in the matrix column, the polymerizable monomer component providing the resin for forming the matrix is described) of the polymerization curable composition and the results of the evaluations are shown in Tables 3 to 5. A radial distribution function graph in the cured product of Reference Comparative Example 2 is shown in Fig. 6. In Reference Comparative Example 2, with a ratio of one out of five tests, a satisfactory evaluation was not obtained. The evaluation results shown in the Tables are based on this system.

As is understood from the results of Reference Examples 1 to 4, it is found that the cured product of the polymerization curable composition provides colored light in a black background and color tone compatibility is satisfactory.

As is understood from the results shown in Figs. 1A, 1B and 2, in the cured product of the polymerization curable composition obtained in Reference Example 1, in the position ($r_1/r_0$ was 1.03) where the closest particle-to-particle distance $r_1$ was 1.03 times the particle diameter $r_0$, the first local maximum peak of the radial distribution function $g(r)$ was observed and the local minimum value of the radial distribution function $g(r)$ between the closest particle-to-particle distance $r_1$ and the second closest particle-to-particle distance $r_2$ was 0.60, showing that the conditions I and II were satisfied.

As is understood from the results shown in Fig. 3, in the cured product of the polymerization curable composition obtained in Reference Example 2, in a position ($r_1/r_0$ was 1.24) where the closest particle-to-particle distance $r_1$ was 1.24 times the particle diameter $r_0$, the first local maximum peak of the radial distribution function $g(r)$ was observed and the local minimum value of the radial distribution function $g(r)$ between the closest particle-to-particle distance $r_1$ and the second closest particle-to-particle distance $r_2$ was 0.62, showing that the conditions I and II were satisfied.

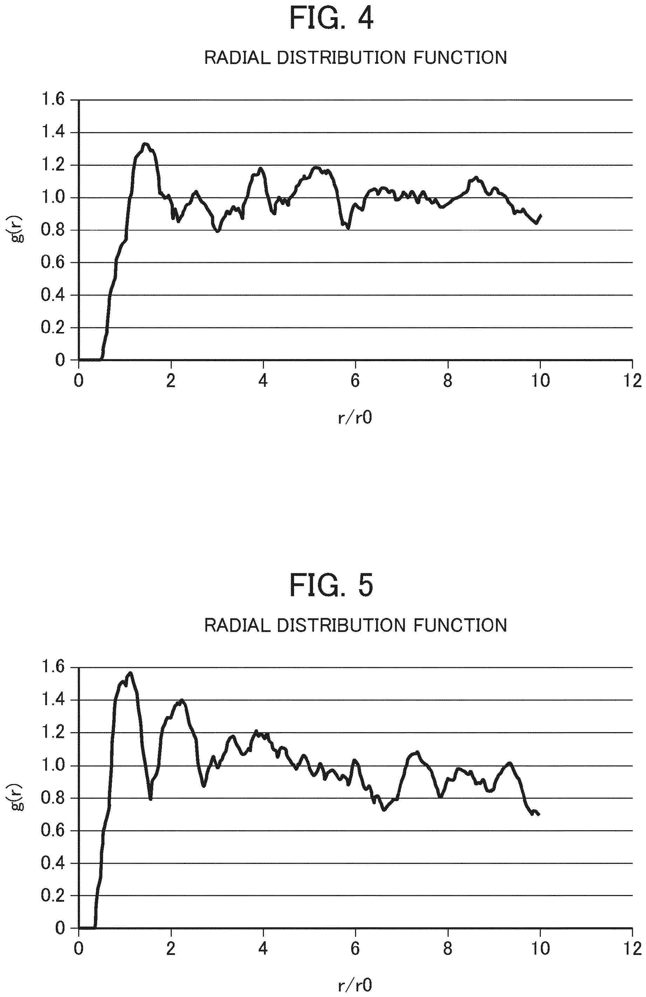

As is understood from the results shown in Fig. 4A, in the cured product of the polymerization curable composition obtained in Reference Example 3, in the position ($r_1/r_0$ was 1.41) where the closest particle-to-particle distance $r_1$ was 1.41 times the particle diameter $r_0$, the first local maximum peak of the radial distribution function $g(r)$ was observed and the local minimum value of the radial distribution function $g(r)$ between the closest particle-to-particle distance $r_1$ and the second closest particle-to-particle distance $r_2$ was 0.88, showing that the conditions I and II were satisfied.

As is understood from the results shown in Fig. 5, in the cured product of the polymerization curable composition obtained in Reference Example 4, in a position ($r_1/r_0$ was 1.04) where the closest particle-to-particle distance $r_1$ was 1.04 times the particle diameter $r_0$, the first local maximum peak of the radial distribution function $g(r)$ was observed and the local minimum value of the radial distribution function $g(r)$ between the closest particle-to-particle distance $r_1$ and the second closest particle-to-particle distance $r_2$ was 0.80, showing that the conditions I and II were satisfied.

As is understood from the results of Reference Comparative Examples 1 and 3 to 5, it is found that in the cured products of the polymerization curable compositions, a desired color tone is not obtained (Reference Comparative Example 1: $n_{(MX)} < n_{(G-PIDm)}$ is not satisfied), colored light is not provided in a black background (Reference Comparative Example 3: the average primary particle diameter of G-PID is 80 nm, Reference Comparative Example 4: the shape of the filler is irregular, Reference Comparative Example 5: a difference of the average primary particle diameters of individual particles of $G\text{-}PID_1$ is less than 25 nm respectively) and color tone compatibility is poor.

As is understood from the results of Reference Comparative Example 2, it is found that when the kneaded state of the composition is non-uniform, color tone compatibility with the tooth substance is poor.

As is understood from the results shown in Fig. 6, in the cured product of the polymerization curable composition obtained in Reference Comparative Example 2, in a position ($r_1/r_0$ is 1.58) where the closest particle-to-particle distance $r_1$ is 1.58 times the particle diameter $r_0$, the first local maximum peak of the radial distribution function $g(r)$ was observed and the local minimum value of the radial distribution function $g(r)$ between the closest particle-to-particle distance $r_1$ and the second closest particle-to-particle distance $r_2$ is 0.18, showing that the condition II is not satisfied.

[Example and Comparative Example]

(Preparation of polymerization curable composition for forming surface exposed layer)

A polymerization curable composition for forming surface exposed layer having a uniform blending composition shown in Table 6 according to Reference Example 1 was prepared using a polymerizable monomer mixture M1 or M2 shown in Table 1, G-PID and G-SFP shown in Table 2, and polymerization initiator components (CQ, DMBE, and HQME). A-1 to A-5 correspond to the first polymerization curable composition (A). A-6 to A-10 do not correspond to the first polymerization curable composition (A). For the cured product of each of the obtained polymerization curable compositions, as in Reference Example 1, (1) the visual evaluation of colored light, (2) the measurement of the wavelength of the colored light, and (3) the evaluation of the radial distribution function of inorganic spherical particles were performed. The results are shown in Table 6.

[Table 6]

| Type | Resin matrix | Identical particle diameter spherical particle group | Superfine particle group | Refractive index difference* | Colored light visual evaluation | Colored light wavelength (nm) Black background | Colored light wavelength (nm) White background | Radial distribution function | |
|---|---|---|---|---|---|---|---|---|---|
| | | | | | | | | Condition I | Condition II |
| A-1 | M1 (100) | G-PID4 (400) | G-SFP (0.5) | 0.006 | yellow | 607 | no local maximum | S | S |
| A-2 | M1 (100) | G-PID5 (200), G-PID8 (200) | G-SFP (1) | 0.006 | red | 695 | no local maximum | s | S |
| A-3 | M1 (100) | G-PID4 (200), G-PID7 (100), G-PID9 (100), G-PID10 (100), G-PID11 (100) | G-SFP (1) | 0.006 | red | 756 | no local maximum | S | S |
| A-4 | M1 (100) | CF1 (300), G-PID5 (200) | G-SFP (1) | 0.006 | red | 720 | no local maximum | S | S |
| A-5 | M1 (100) | G-PID4 (400) | G-SFP (0) | 0.006 | yellow | 607 | no local maximum | s | S |
| A-6 | M2 (100) | G-PID4 (400) | G-SFP (0.5) | -0.031 | blue | 481 | no local maximum | - | - |
| A-7 | M1 (100) | G-PID2 (400) | G-SFP (0.5) | 0.006 | dark blue | 430 | no local maximum | s | N |
| A-8 | M1 (100) | G-PID1 (400) | G-SFP (0.5) | 0.006 | none | 405 | no local maximum | - | - |
| A-9 | M1 (100) | F1 (400) | G-SFP (0.5) | 0.006 | none | no local maximum | no local maximum | - | - |

(continued)

| Type | Resin matrix | Identical particle diameter spherical particle group | Superfine particle group | Refractive index difference* | Colored light visual evaluation | Colored light wavelength (nm) Black background | Colored light wavelength (nm) White background | Radial distribution function | |
|---|---|---|---|---|---|---|---|---|---|
| | | | | | | | | Condition I | Condition II |
| A-10 | M1 (100) | G-PID3 (100), G-PID4 (200), G-PID5 (200), G-PID6 (100) | G-SFP (1) | 0.006 | none | no local maximum | no local maximum | - | - |

*Refractive index of identical particle diameter spherical particle group (G-PID) - refractive index of polymer of resin matrix

(Preparation of polymerization curable composition for forming foundation layer)

To 100 parts by mass of polymerizable monomer component M2, 0.3 parts by mass of CQ, 1.0 parts by mass of DMBE, and 0.15 parts by mass of HQME were added and mixed to prepare a uniform polymerizable monomer composition. Next, in a mortar, 75 parts by mass of PF-1 was weighed, and the polymerizable monomer composition was gradually added under reddish light so that the blending amount of M2 was 25 parts by mass, the mixture was kneaded sufficiently in a dark place, and then white pigment, yellow pigment, red pigment, and blue pigment were added in an amount shown in Table 7, and kneaded to obtain a uniform curable paste. Furthermore, the paste was defoamed under reduced pressure to remove air bubbles, thereby producing polymerization curable compositions for forming foundation layer: B-1 to B-6. The value of the cured product of the obtained polymerization curable composition in a black background was evaluated as follows. In other words, paste of the prepared polymerization curable composition was put into a mold having a through-hole of 7 mm$\phi \times$ 1 mm, and polyester films were pressed on both sides. Curing was performed by light application for 30 seconds on both sides using the visible light irradiator (Power Light, manufactured by Tokuyama Corporation), and thereafter the cured product was taken out of the mold, and value (V) was measured using a color difference meter ("TC-1800MKII", manufactured by Tokyo Denshoku Co., Ltd.). The results are shown in Table 7. As shown in Table 7, B-2 to B-6 correspond to the second polymerization curable composition (B), and B-1 does not correspond to the second polymerization curable composition (B).

[Table 7]

| Type | Pigment/part by mass | | | | Value (V) |
|---|---|---|---|---|---|
| | White pigment | Yellow pigment | Red pigment | Blue pigment | |
| B-1 | 0.01523 | 0.00023 | 0.00021 | 0.00006 | 4.2 |
| B-2 | 0.03015 | 0.00035 | 0.00029 | 0.00010 | 5.8 |
| B-3 | 0.04422 | 0.00055 | 0.00032 | 0.00010 | 6.1 |
| B-4 | 0.05092 | 0.00075 | 0.00030 | 0.00009 | 6.6 |
| B-5 | 0.08375 | 0.00125 | 0.00045 | 0.00021 | 7.3 |
| B-6 | 0.11050 | 0.00145 | 0.00052 | 0.00030 | 8.0 |

<Examples 1 to 10, Comparative Examples 1 to 6>

Restoration of simulated cavity was performed using the polymerization curable composition for forming a foundation layer shown in Table 8 and the polymerization curable composition for forming surface exposed layer, and the color tone compatibility was evaluated. The results are shown in Table 8.

(1) Evaluation of color tone compatibility

The following simulated restorations were performed using a model tooth for tooth restoration that reproduced the class IV cavity (depth 5 mm, height 7 mm, width 4 mm) in the upper right number 1. In other words, firstly, a polymerization curable composition for forming a foundation layer was filled in a lost portion at a tongue side to a thickness of 0.5 mm, and cured by irradiation with light using a visible light irradiator (Power Light manufactured by Tokuyama Corporation) for 30 seconds to form a foundation layer. Then, a polymerization curable composition for forming surface exposed layer was laminated by filling on the formed foundation layer to form a lost portion in a form of tooth, followed by being irradiated with light using the visible light irradiator (Power Light, manufactured by Tokuyama Corporation) for 30 seconds and curing thereof to form a surface exposed layer. Thereafter, the filled was polished, and thus the simulated restoration of the lost portion was completed. Then, the color tone compatibility was checked by visual observation, and evaluated according to the below-mentioned criteria. As the model tooth for teeth restoration, a high value model tooth and low value model tooth in the class of A2 (reddish brown) in a shade guide ("VITA Classical", manufactured by Vita Zahnfabrik H. Rauter GmbH & Co. KG).

-Criteria-

A: Color tone of restored product has good compatibility with a model tooth for tooth restoration.
B: Color tone of restored product is similar to that of a model tooth for tooth restoration.
C: Color tone of restored product is similar to that of a model tooth for tooth restoration but does not have good compatibility.
D: Color tone of restored product does not have compatibility with a model tooth for tooth restoration.

[Table 8]

| No. | | Polymerization curable composition for forming surface exposed layer | Polymerization curable composition for forming foundation layer | | Color tone compatibility (low value model tooth) Corresponds to A2 | Color tone compatibility (high value model tooth) Corresponds to A2 |
|---|---|---|---|---|---|---|
| | | | Type | Value (V) | | |
| Example | 1 | A-1 | B-2 | 5.8 | A | B |
| | | A-1 | B-3 | 6.1 | A | A |
| | 3 | A-1 | B-4 | 6.6 | A | A |
| | 4 | A-1 | B-5 | 7.3 | A | A |
| | 2 5 | A-1 | B-6 | 8.0 | B | A |
| | 6 | A-1 | B-5 | 7.3 | A | A |
| | 7 | A-2 | B-5 | 7.3 | A | A |
| | 8 | A-3 | B-5 | 7.3 | A | A |
| | 9 | A-4 | B-5 | 7.3 | A | A |
| | 10 | A-5 | B-5 | 7.3 | A | A |
| Comparative Example | 1 | A-1 | B-1 | 4.2 | C | D |
| | 2 | A-6 | B-5 | 7.3 | D | D |
| | 3 | A-7 | B-5 | 7.3 | D | D |
| | 4 | A-8 | B-5 | 7.3 | D | D |
| | 5 | A-9 | B-5 | 7.3 | D | D |
| | 6 | A-10 | B-5 | 7.3 | D | D |

As shown in Table 8, in Examples 1 to 10 in which the first polymerization curable compositions (A): A-1 to A-5 were used as the polymerization curable composition for forming surface exposed layer, and the second polymerization curable compositions (B): B-2 to B-6 were used as the polymerization curable composition for forming a foundation layer, the surface exposed layer showed yellow-red colored light due to interference of light under a black background, and the color compatibility was good, showing good color tone compatibility. On the contrary, in Comparative Example 1 in which a polymerization curable composition for forming a foundation layer: B-1 having the value of the cured product of 4.2 was used, although the first polymerization curable composition (A) was used as the polymerization curable composition for forming surface exposed layer, the color tone compatibility was low. Furthermore, in Comparative Examples 2 to 6 in which polymerization curable composition for forming surface exposed layers: A-6 to A-10 not corresponding to the first polymerization curable composition (A) were used, although the second polymerization curable composition (B): B-5 having value of the cured product as the polymerization curable composition for forming a foundation layer of 7.3 was used, the color tone compatibility was low.

**Claims**

1. A dental filling restorative material kit for restoring a tooth having at least one of a cavity of class III and a cavity of class IV, the kit comprising:

    a first polymerization curable composition (A) for forming a surface exposed layer to be exposed to a surface after restoration, and a second polymerization curable composition (B) for forming a foundation layer serving as a foundation of the surface exposed layer,
    wherein the first polymerization curable composition (A) satisfies the following (a1) to (a3):

       (a1) the first polymerization curable composition (A) comprises a first polymerizable monomer component, inorganic particles, and a first polymerization initiator component;
       wherein the refractive index of the first polymerizable monomer component is 1.38 to 1.55, at 25°C with respect to light of a wavelength of 589 nm;
       (a2) the inorganic particles satisfy all of following conditions (i) to (iii):

          (i) the inorganic particles comprise an identical particle diameter spherical particle group (G-PID) which is formed of an aggregate of inorganic spherical particles having a predetermined average primary particle diameter within a range of 100 nm to 1000 nm and in which 90% or more of all the particles are present within a range of plus or minus 5% of the predetermined average primary particle diameter, in a number-based particle size distribution of the aggregate, and the number of the identical particle diameter spherical particle groups included in the inorganic particles is one or more, and wherein the inorganic spherical particles are silica-titanium group element oxide-based composite oxide particles having a refractive index of 1.45 to 1.58, at 25°C with respect to light of a wavelength of 589 nm;
          (ii) when the number of the identical particle diameter spherical particle groups included in the inorganic particles is represented as a, and each of the identical particle diameter spherical particle groups is represented as $G\text{-}PID_1$ (when a is 1, m is 1, and when a is 2 or more, m is a natural number from 1 to a) in ascending order of the average primary particle diameters thereof, the average primary particle diameters of each $G\text{-}PID_1$ differ from each other by 25 nm or more and wherein the total content of the identical particle diameter spherical particle group is 10 to 1500 parts by mass with respect to 100 parts by mass of the first polymerizable monomer component; and
          (iii) when a refractive index of a cured product of the first polymerizable monomer component at 25°C with respect to light of a wavelength of 589 nm is represented as $n_{(MX)}$, and a refractive index of the inorganic spherical particles constituting each $G\text{-}PID_1$ at 25°C with respect to light of a wavelength of 589 nm is represented as $n_{(G\text{-}PIDm)}$, for any $n_{(G\text{-}PIDm)}$ a relationship of $n_{(MX)} < n_{(G\text{-}PIDm}$ is satisfied; and

       (a3) in a cured product (A') obtained by curing the first polymerization curable composition (A),

          when a radial distribution function representing a probability of finding an inorganic spherical particle at a distance of r away from a center of a given inorganic spherical particle is defined by the following formula (1),

$$g(r) = \{1/<\rho>\} \times \{dn/da\} \quad (1),$$

          wherein $<\rho>$ represents an average particle density of the inorganic spherical particles in an observation plane,
          dn represents the number of the inorganic spherical particles being present in a region between a circle at a distance of r and r + dr away from a given inorganic spherical particle within the observation plane, da represents an area of the region (da = $2\pi r \cdot dr$), and

$\langle\rho\rangle$, dn, and da are determined based on a scanning electron microscope image in which a plane in the cured product (A') is the observation plane, and

a radial distribution function graph representing a relationship between $r/r_0$ and g(r) corresponding to r at that time is created by plotting $r/r_0$ on x-axis and g(r) on y-axis, wherein $r/r_0$ is a dimensionless number standardized by dividing a distance r away from a center of a given inorganic spherical particle dispersed in the cured product (A') by an average particle diameter $r_0$ of all the inorganic spherical particles dispersed in the cured product (A'),

the inorganic spherical particles are dispersed in the first polymerization curable composition (A) such that the cured product (A') has a short-range order structure satisfying the following conditions (I) and (II):

(I) a closest particle-to-particle distance $r_1$ defined as r corresponding to a peak top of a peak closest to an origin among peaks appearing in the radial distribution function graph is a value being 1 to 2 times the average particle diameter $r_0$ of all the inorganic spherical particles dispersed in the cured product; and

(II) when r corresponding to a peak top of a peak second closest to the origin among the peaks appearing in the radial distribution function graph is represented as a second closest particle-to-particle distance $r_2$, a local minimum value of the radial distribution function g(r) between the closest particle-to-particle distance $r_1$ and the second closest particle-to-particle distance $r_2$ is a value of 0.56 to 1.10, and

wherein the second polymerization curable composition (B) satisfies the following (b1) and (b2):

(b1) the second polymerization curable composition (B) comprises a second polymerizable monomer component, a colorant component, and a second polymerization initiator component, and

(b2) the colorant component is blended such that value (V) of a colorimetric value by a Munsell color system of colored light measured using a color-difference meter in a black background is 5 or more with respect to a sample comprising the cured product (B') of the second polymerization curable composition (B) and having a thickness of 1 mm.

2. The dental filling restorative material kit according to claim 1, wherein the first polymerization curable composition (A) further comprises a superfine particle group (G-SFP) having an average primary particle diameter of less than 100 nm, and having an average primary particle diameter being smaller than the average primary particle diameter of G-PID$_1$ by 25 nm or more, as the inorganic particles.

3. The dental filling restorative material kit according to claim 2, wherein the first polymerization curable composition (A) does not substantially comprise a colorant component other than the first polymerizable monomer component, the first polymerization initiator component, the identical particle diameter spherical particle group, and the superfine particle group.

4. The dental filling restorative material kit according to any one of claims 1 to 3, wherein the first polymerization curable composition (A) comprises only one type of identical particle diameter spherical particle group having an average primary particle diameter of the inorganic spherical particles within a range from 230 nm to 350 nm, as the identical particle diameter spherical particle group G-PID.

**Patentansprüche**

1. Set aus einem zahnärztlichen Füll- und Sanierungsmaterial zur Sanierung eines Zahns, der eine Kavität der Klasse III und/oder eine Kavität der Klasse IV hat, wobei das Set aufweist:

eine erste durch Polymerisation härtbare Zusammensetzung (A) zur Bildung einer exponierten Oberflächenschicht, die nach Sanierung zu einer Oberfläche hin freiliegen soll, und eine zweite durch Polymerisation härtbare Zusammensetzung (B) zur Bildung einer Grundschicht, die als Fundament der exponierten Oberflächenschicht dient,

wobei die erste durch Polymerisation härtbare Zusammensetzung (A) die folgenden Bedingungen (a1) bis (a3) erfüllt:

(a1) die erste durch Polymerisation härtbare Zusammensetzung (A) weist eine erste polymerisationsfähige Monomerkomponente, anorganische Partikel und eine erste Polymerisations-Initiatorkomponente auf; wobei der Brechungsindex der ersten polymerisationsfähigen Monomerkomponente bei 25 °C in Bezug auf Licht mit einer Wellenlänge von 589 nm 1,38 bis 1,55 beträgt;

(a2) die anorganischen Partikel erfüllen sämtliche der folgenden Bedingungen (i) bis (iii):

(i) die anorganischen Partikel weisen eine Gruppe (G-PID) aus sphärischen Partikeln mit identischem Partikeldurchmesser auf, die aus einer Ansammlung anorganischer sphärischer Partikel mit einem vorbestimmten mittleren primären Partikeldurchmesser in einem Bereich von 100 nm bis 1000 nm gebildet ist und bei denen in einer zahlenbasierten Partikelgrößenverteilung der Ansammlung 90 % oder mehr aller Partikel in einem Bereich von plus oder minus 5 % des vorbestimmten mittleren primären Partikeldurchmessers liegen, und die Anzahl der Gruppen aus sphärischen Partikeln mit identischem Partikeldurchmesser, die in den anorganischen Partikeln enthalten sind, eins oder mehr beträgt, und wobei es sich bei den anorganischen sphärischen Partikeln um Verbundoxidpartikel auf der Basis eines Siliziumdioxid-Titan-Gruppenelement-Oxids mit einem Brechungsindex von 1,45 bis 1,58 bei 25 °C in Bezug auf eine Licht einer Wellenlänge von 589 nm handelt;

(ii) wenn die Anzahl der Gruppen aus sphärischen Partikeln mit identischem Partikeldurchmesser, die in den anorganischen Partikeln enthalten sind, durch a dargestellt wird, und jede der Gruppen aus sphärischen Partikeln mit identischem Partikeldurchmesser durch $G\text{-}PID_m$ dargestellt wird (wenn a gleich 1 ist, ist m gleich 1, und wenn a größer oder gleich 2 ist, ist m eine natürliche Anzahl von 1 bis a), in aufsteigender Reihenfolge der mittleren primären Partikeldurchmesser von diesen, unterscheiden sich die mittleren primären Partikeldurchmesser von jeder $G\text{-}PID_m$ um 25 nm oder mehr voneinander, und wobei der Gesamtanteil der Gruppe aus sphärischen Partikeln mit identischem Partikeldurchmesser 10 bis 1500 Massenteile in Bezug auf 100 Massenteile der ersten polymerisationsfähigen Monomerkomponente beträgt; und

(iii) wenn ein Brechungsindex eines ausgehärteten Produkts der ersten polymerisationsfähigen Monomerkomponente bei 25°C in Bezug auf Licht einer Wellenlänge von 589 nm durch $n_{(MX)}$ dargestellt wird und ein Brechungsindex der anorganischen sphärischen Partikel, die eine jeweilige $G\text{-}PID_m$ bilden, bei 25°C in Bezug auf Licht einer Wellenlänge von 589 nm durch $n_{(G\text{-}PIDm)}$ dargestellt wird, ist für jeden Index $n_{(G\text{-}PIDm)}$ die Beziehung $n_{(MX)} < n_{(G\text{-}PIDm)}$ erfüllt; und

(a3) in einem ausgehärteten Produkt (A'), das erhalten wird, indem die erste durch Polymerisation härtbare Zusammensetzung (A) ausgehärtet wird,

wenn eine Radialverteilungsfunktion, die eine Wahrscheinlichkeit angibt, dass ein anorganisches sphärisches Partikel unter einem Abstand r von der Mitte eines bestimmten anorganischen sphärischen Partikels entfernt ist, durch folgende Formel (1) definiert ist,

$$g(r) = \{1/<\rho>\} \times \{dn/da\} \quad (1),$$

wobei $<\rho>$ eine mittlere Partikeldichte der anorganischen sphärischen Partikel in einer Beobachtungsebene darstellt,

dn die Anzahl der anorganischen sphärischen Partikel darstellt, die in einem Bereich zwischen einem Kreis mit einem Abstand r und r + dr von einem bestimmten anorganischen sphärischen Partikel innerhalb der Beobachtungsebene vorhanden sind,

da eine Fläche des Bereichs darstellt (da = 2πr · dr) und $<\rho>$, dn und da beruhend auf einer Rasterelektronenmikroskop-Aufnahme bestimmt werden, in der eine Ebene in dem ausgehärteten Produkt (A') die Beobachtungsebene ist, und

ein Radialverteilungsfunktions-Diagramm, das eine Beziehung zwischen $r/r_0$ und g(r) entsprechend r zu diesem Zeitpunkt darstellt, geschaffen wird, indem man $r/r_0$ auf der x-Achse und g(r) auf der y-Achse aufträgt, wobei $r/r_0$ eine dimensionslose Zahl ist, die standardisiert wird, indem ein Abstand r von der Mitte eines bestimmten anorganischen sphärischen Partikels, das in dem ausgehärteten Produkt (A') verteilt ist, durch einen mittleren Partikeldurchmesser $r_0$ aller anorganischen sphärischen Partikel, die in dem ausgehärteten Produkt (A') verteilt sind, dividiert wird,

die anorganischen sphärischen Partikel in der ersten durch Polymerisation härtbaren Zusammensetzung (A) so verteilt sind, dass das ausgehärtete Produkt (A') eine Nahbereichs-Ordnungsstruktur hat, die die folgenden Bedingungen (I) und (II) erfüllt:

(I) ein engster Partikel-zu-Partikel-Abstand $r_1$, der als r entsprechend einem Spitzenwert eines Peaks definiert ist, der unter den in dem Radialverteilungsfunktions-Diagramm erscheinenden Peaks dem Ursprung am nächsten ist, ist ein Wert, der dem 1- bis 2-fachen des mittleren Partikeldurchmessers $r_0$ aller anorganischen sphärischen Partikel entspricht, die in dem ausgehärteten Produkt verteilt sind; und

(II) wenn r entsprechend einem Spitzenwert eines Peaks, der unter den in dem Radialverteilungsfunktions-Diagramm erscheinenden Peaks dem Ursprung am zweitnächsten ist, als zweitengster Partikel-zu-Partikel-Abstand $r_2$ angenommen wird, ist ein lokaler Mindestwert der Radialverteilungsfunktion g(r) zwischen dem engsten Partikel-zu-Partikel-Abstand $r_1$ und dem zweitengsten Partikel-zu-Partikel-Abstand $r_2$ ein Wert von 0,56 bis 1,10, und

wobei die zweite durch Polymerisation härtbare Zusammensetzung (B) die folgenden Bedingungen (b1) und (b2) erfüllt:

(b1) die zweite durch Polymerisation härtbare Zusammensetzung (B) weist eine zweite polymerisationsfähige Monomerkomponente, ein Farbstoffkomponente und eine zweite Polymerisations-Initiatorkomponente auf, und

(b2) die Farbstoffkomponente ist so beigemischt, dass ein Wert (V) eines kolorimetrischen Werts nach einem Munsell-Farbsystem aus farbigem Licht, der mithilfe einer Farbdifferenz-Messvorrichtung vor schwarzem Hintergrund gemessen wird, gleich 5 oder mehr in Bezug auf eine Probe hat, die das ausgehärtete Produkt (B') der zweiten durch Polymerisation härtbaren Zusammensetzung (B) aufweist und eine Dicke von 1 mm hat.

2. Set aus einem zahnärztlichen Füll- und Sanierungsmaterial nach Anspruch 1, wobei die erste durch Polymerisation härtbare Zusammensetzung (A) darüber hinaus eine Gruppe (G-SFP) aus superfeinen Partikeln aufweist, die einen mittleren primären Partikeldurchmesser von unter 100 nm hat, und einen mittleren primären Partikeldurchmesser hat, der um 25 nm oder mehr kleiner ist als der mittlere primäre Partikeldurchmesser von G-PID$_1$, als die anorganischen Partikel.

3. Set aus einem zahnärztlichen Füll- und Sanierungsmaterial nach Anspruch 2, wobei die erste durch Polymerisation härtbare Zusammensetzung (A) im Wesentlichen keine andere Farbstoffkomponente als die erste polymerisationsfähige Monomerkomponente, die erste Polymerisations-Initiatorkomponente, die Gruppe aus sphärischen Partikeln mit identischem Partikeldurchmesser und die Gruppe aus superfeinen Partikeln aufweist.

4. Set aus einem zahnärztlichen Füll- und Sanierungsmaterial nach einem der Ansprüche 1 bis 3, wobei die erste durch Polymerisation härtbare Zusammensetzung (A) nur eine Art einer Gruppe aus sphärischen Partikeln mit identischem Partikeldurchmesser mit einem mittleren primären Partikeldurchmesser der anorganischen sphärischen Partikel in einem Bereich von 230 nm bis 350 nm aufweist, als die Gruppe G-PID aus sphärischen Partikeln mit identischem Partikeldurchmesser.

**Revendications**

1. Kit de matériaux d'obturation et de réparation dentaire destiné à réparer une dent présentant au moins l'une d'une cavité de classe III et d'une cavité de classe IV, le kit comprenant :

une première composition durcissable par polymérisation (A) permettant de former une couche exposée en surface devant être exposée vers une surface après la réparation, et une seconde composition durcissable par polymérisation (B) permettant de former une couche de fond servant de fond à la couche exposée en surface, sachant que la première composition durcissable par polymérisation (A) satisfait aux termes (a1) à (a3) suivants :

(a1) la première composition durcissable par polymérisation (A) comprend un premier composant monomère polymérisable, des particules inorganiques, et un premier composant initiateur de polymérisation ; sachant que l'indice de réfraction du premier composant monomère polymérisable est de 1,38 à 1,55, à 25 °C par rapport à une lumière d'une longueur d'onde de 589 nm ;

(a2) les particules inorganiques satisfont à la totalité des conditions (i) à (iii) suivantes :

(i) les particules inorganiques comprennent un groupe de particules sphériques de diamètre de particule identique (G-PID) qui est composé d'un aggrégat de particules sphériques inorganiques ayant un diamètre de particule primaire moyen prédéterminé compris dans une plage de 100 nm à 1000 nm et dans lequel 90 % ou plus de toutes les particules sont présentes dans une plage de plus ou moins 5 % du diamètre de particule primaire moyen prédéterminé, dans une distribution de tailles de particule de l'agrégat basée sur le nombre, et le nombre des groupes de particules sphériques de diamètre de particule identique inclus dans les particules inorganiques est de un ou plus, et sachant que les particules sphériques inorganiques sont des particules d'oxyde composite basées sur un élément d'oxyde du groupe silice-titane ayant un indice de réfraction de 1,45 à 1,58, à 25 °C par rapport à une lumière d'une longueur d'onde de 589 nm ;

(ii) lorsque le nombre de groupes de particules sphériques de diamètre de particule identique inclus dans les particules inorganiques est représenté comme a, et chacun des groupes de particules sphériques de diamètre de particule identique est représenté comme $G\text{-}PID_m$ (lorsque a est 1, m est 1, et lorsque a est 2 ou plus, m est un nombre naturel de 1 à a) par ordre croissant des diamètres de particule primaires moyens de ceux-ci, les diamètres de particule primaires moyens de chaque $G\text{-}PID_m$ diffèrent les uns des autres à raison de 25 nm ou plus et sachant que la teneur totale du groupe de particules sphériques de diamètre de particule identique est de 10 à 1500 parties en masse par rapport à 100 parties en masse du premier composant monomère polymérisable ; et

(iii) lorsqu'un indice de réfraction d'un produit durci du premier composant monomère polymérisable à 25 °C par rapport à une lumière d'une longueur d'onde de 589 nm est représenté comme $n_{(MX)}$, et un indice de réfraction des particules sphériques inorganiques constituant chaque $G\text{-}PID_m$ à 25 °C par rapport à une lumière d'une longueur d'onde de 589 nm est représenté comme $n_{(G\text{-}PIDm)}$, pour n'importe quel $n_{(G\text{-}PIDm)}$, une relation de $n_{(MX)} < n_{(G\text{-}PIDm)}$ est satisfaite ; et

(a3) dans un produit durci (A') obtenu en durcissant la première composition durcissable par polymérisation (A),

lorsqu'une fonction de distribution radiale représentant une probabilité de trouver une particule sphérique inorganique à une distance de r d'un centre d'une particule sphérique inorganique donnée est définie par la formule (1) suivante,

$$g(r) = \{1/\langle \rho \rangle\} \times \{dn/da\} \quad (1),$$

sachant que $\langle \rho \rangle$ représente une densité de particule moyenne des particules sphériques inorganiques dans un plan d'observation,

dn représente le nombre des particules sphériques inorganiques qui sont présentes dans une région au sein d'un cercle à une distance de r et r + dr d'une particule sphérique inorganique donnée au sein du plan d'observation,

da représente une aire de la région (da = 2nr · dr), et $\langle \rho \rangle$, dn, et da sont déterminés sur la base d'une image par microscope électronique à balayage dans laquelle un plan dans le produit durci (A') est le plan d'observation, et

un graphe de fonction de distribution radiale représentant une relation entre $r/r_0$ et g(r) correspondant à r à ce moment est créé en traçant $r/r_0$ sur l'axe x et g(r) sur l'axe y, sachant que $r/r_0$ est un nombre sans dimension standardisé en divisant une distance r par rapport à un centre d'une particule sphérique inorganique donnée dispersée dans le produit durci (A') par un diamètre de particule moyen $r_0$ de toutes les particules sphériques inorganiques dispersées dans le produit durci (A'),

les particules sphériques inorganiques sont dispersées dans la première composition durcissable par polymérisation (A) de telle sorte que le produit durci (A') ait une structure d'ordre à courte portée satisfaisant aux conditions (I) et (II) suivantes :

(I) une distance de particule à particule la plus proche $r_1$ définie comme r correspondant à un sommet de pic d'un pic le plus proche d'une origine parmi des pics apparaissant dans le graphe de fonction de distribution radiale est une valeur qui est de 1 à 2 fois le diamètre de particule moyen $r_0$ de toutes les particules sphériques inorganiques dispersées dans le produit durci ; et

(II) lorsque r correspondant à un sommet de pic d'un pic le second plus proche de l'origine parmi les pics apparaissant dans le graphe de fonction de distribution radiale est représenté comme une distance de particule à seconde particule la plus proche $r_2$, une valeur minimale locale de la fonction de distribution radiale g(r) entre la distance de particule à particule la plus proche $r_1$ et la distance de particule à seconde particule la plus proche $r_2$ est une valeur de 0,56 à 1,10, et

sachant que la seconde composition durcissable par polymérisation (B) satisfait aux termes (b1) et (b2) suivants :

(b1) la seconde composition durcissable par polymérisation (B) comprend un second composant monomère polymérisable, un composant colorant, et un second composant initiateur de polymérisation, et

(b2) le composant colorant est mélangé de telle sorte qu'une valeur (V) d'une valeur colorimétrique selon un nuancier de Munsell de lumière colorée mesurée à l'aide d'un dispositif de mesure de différence de couleur sur fond noir soit de 5 ou plus par rapport à un échantillon comprenant le produit durci (B') de la seconde composition durcissable par polymérisation (B) et ayant une épaisseur de 1 mm.

2. Le kit de matériaux d'obturation et de réparation dentaire selon la revendication 1, sachant que la première composition durcissable par polymérisation (A) comprend en outre un groupe de particules superfines (G-SFP) ayant un diamètre de particule primaire moyen de moins de 100 nm, et ayant un diamètre de particule primaire moyen qui est inférieur au diamètre de particule primaire moyen de G-PID$_1$ à raison de 25 nm ou plus, en tant que les particules inorganiques.

3. Le kit de matériaux d'obturation et de réparation dentaire selon la revendication 2, sachant que la première composition durcissable par polymérisation (A) ne comprend sensiblement pas de composant colorant autre que le premier composant monomère polymérisable, le premier composant initiateur de polymérisation, le groupe de particules sphériques de diamètre de particule identique, et le groupe de particules superfines.

4. Le kit de matériaux d'obturation et de réparation dentaire selon l'une quelconque des revendications 1 à 3, sachant que la première composition durcissable par polymérisation (A) comprend seulement un type de groupe de particules sphériques de diamètre de particule identique ayant un diamètre de particule primaire moyen des particules sphériques inorganiques compris dans une plage de 230 nm à 350 nm, en tant que le groupe de particules sphériques de diamètre de particule identique G-PID.

# FIG. 1A

# FIG. 1B

# FIG. 2

## RADIAL DISTRIBUTION FUNCTION

# FIG. 3

## RADIAL DISTRIBUTION FUNCTION

# FIG. 4

RADIAL DISTRIBUTION FUNCTION

# FIG. 5

RADIAL DISTRIBUTION FUNCTION

# FIG. 6

## RADIAL DISTRIBUTION FUNCTION

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 5436723 B
- JP 6258919 B
- JP 5274164 B
- JP 6250245 B
- JP 6732257 B
- JP 6732259 B
- WO 2011115007 A
- WO 2013039169 A
- JP 58110414 A
- JP 58156524 A
- JP 2132102 A
- JP 3197311 A

### Non-patent literature cited in the description

- Adhesion YEARBOOK 2006. Quintessence Publishing Co., August 2006, 129-137
- **MASASHI MIYAZAKI**. Science & Technique of Composite Resin Restoration. Quintessence Publishing Co., January 2010, 48-49